# FASCICULE DE BREVET EUROPEEN

(11) **EP 0 998 470 B1**
(45) Date de publication et mention de la délivrance du brevet: **07.05.2003**
(21) Numéro de dépôt: 98937591.0
(22) Date de dépôt: 09.07.1998
(51) Int. Cl.: C07D 307/33, C07K 5/10, A61K 38/07, A61K 31/34

(54) **PROCEDE DE PREPARATION DE DERIVES AMINES D'ALKYLOXYFURANONE, COMPOSES ISSUS DE CE PROCEDE ET UTILISATION DE CES COMPOSES**
VERFAHREN ZUR HERSTELLUNG VON ALKOXYFURANON-AMIN-DERIVATEN, DURCH DIESES VERFAHREN ERHALTENE VERBINDUNGEN UND VERWENDUNG DIESER VERBINDUNGEN
METHOD FOR PREPARING ALKYLOXY FURANONE DERIVATIVES, COMPOUNDS OBTAINED BY SAID METHOD AND USE OF SAID COMPOUNDS

(30) Priorité: 15.07.1997 FR 9708932
(43) Date de publication de la demande: 10.05.2000
(73) Titulaire: Aventis Pharma S.A., 92160 Antony (FR)
(72) Inventeur: BOUCHET, Raphael, F-93500 Pantin (FR); BRION, Francis, F-31500 Toulouse (FR); COLLADANT, Colette, F-93110 Rosny-sous-Bois (FR); LAGOUARDAT, Jacques, F-93160 Noisy-le-Grand (FR)
(86) Numéro de dépôt international: FR9801480
(87) Numéro de publication internationale: WO99003852

(56) Documents cités:
- WO-A-95/35308
- WO-A-97/22619
- CHAPMAN K T: "Synthesis of a potent, reversible inhibitor of interleukin-1.beta. converting enzyme" BIOORG. MED. CHEM. LETT. (BMCLE8,0960894X);92; VOL.2 (6); PP.613-18, XP000570540 cité dans la demande
- FABER W S ET AL: "Catalytic kinetic resolution of 5-alkoxy-2(5H)-furanones" TETRAHEDRON (TETRAB,00404020);94; VOL.50 (16); PP.4775-94, XP002060186 UNIV. GRONINGEN;DEP. ORG. MOL. INORG. CHEM.; GRONINGEN; 9747 AG; NETH. (NL)
- DE LANGE B ET AL: "Asymmetric 1,4-additions to 5-alkoxy-2(5H)-furanones. Enantioselective synthesis and absolute configuration determination of.beta.-amino-.gamma.-butyrolactones and amino diols" TETRAHEDRON (TETRAB,00404020);89; VOL.45 (21); PP.6799-818, XP002060187
- FERINGA B L ET AL: "Asymmetric synthesis of 2-amino-1,4-diols" TETRAHEDRON LETT. (TELEAY,00404039);88; VOL.29 (11); PP.1303-6, XP002060188

## Description

La présente invention a pour objet un nouveau procédé de préparation de dérivés aminés d'alkyloxyfuranone, les composés issus de ce procédé et l'utilisation de ces composés dans la synthèse d'inhibiteurs de l'enzyme de conversion de l'interleukine-1bêta.

Les demandes de brevets WO9535308, WO9722619, WO9722618, EP519748, WO9633209 décrivent des composés inhibiteurs de l'enzyme de conversion de l'interleukine-1bêta.

Le procédé de préparation de certains des composés décrits dans les demandes de brevets citées plus haut met en oeuvre les composés de formule (I) suivants 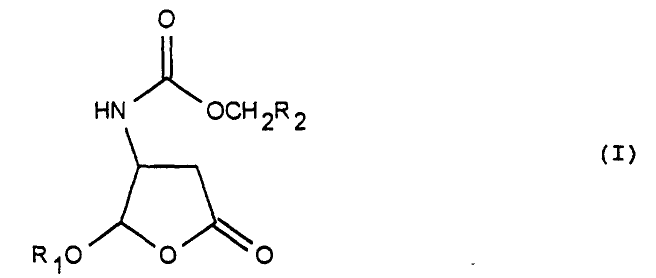 dans laquelle R₁ représente un radical éthyle et R₂ représente un radical -CH=CH₂.

Les composés de formule (I) sont préparés à partir d'acide L-aspartique protégé et nécessitent 4 étapes de synthèse : 1) acylation, 2) réduction, 3) oxydation, 4) cyclisation, (Chapman K.T. et al Bioorg. Med. Chem. Lett. 2(6), 613-8 (1992)).

Ce procédé présente des inconvénients importants, notamment dans le cas où l'on désire obtenir des composés de formule (I) chiraux. Il faut en effet mettre en oeuvre un réactif chiral de départ coûteux : l'acide L-aspartique β-tert-butylester, et surtout utiliser des méthodes chromatographiques pour isoler et/ou purifier les différents diastéréoisomères.

La présente invention a donc pour objet de trouver une autre voie de synthèse des composés de formule (I), qui évite de partir de ce produit de départ et qui ne nécessite pas de séparations par chromatographie.

La demanderesse propose donc une nouvelle voie de synthèse, à partir d'alkyloxyfuranone de formule (II), sous forme racémique, permettant d'avoir accès aux composés nouveaux de formules (IVa), (IVb), (IVc) ou (IVd) telles que définies plus bas, salifiés ou non salifiés, qui sont par la suite, le cas échéant protégés, afin d'obtenir notamment les composés de formule (I).

Ce procédé présente l'avantage de pouvoir être mis en oeuvre à grande échelle, à partir d'un composé de formule (II), aisément accessible et peu coûteux, avec les étapes de séparation et/ou de purification s'effectuant par cristallisation et non par chromatographie. Chaque diastéréoisomère de formule (III), (IV) ou (I) peut ainsi être isolé.

La présente invention a donc d'une part pour objet des composés de formule (IVa), (IVb), (IVc) ou (IVd) suivants : 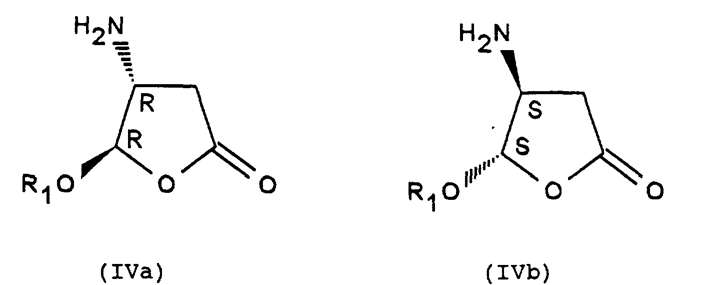 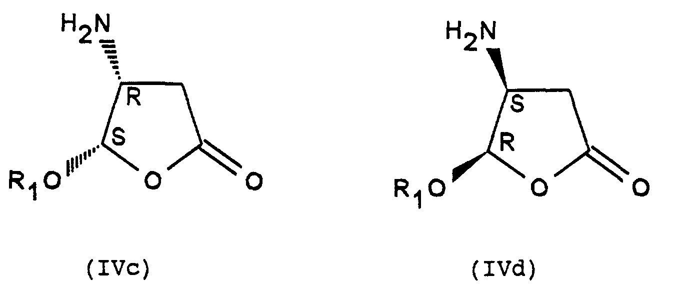 dans lesquelles R₁ est un groupement alkyle renfermant de 1 à 4 atomes de carbone ou un groupement phénylalkyle renfermant de 7 à 11 atomes de carbone, ainsi que leurs sels d'addition avec les acides.

L'invention s'étend naturellement aux sels des composés de formule (IVa), (IVb), (IVc) ou (IVd), comme par exemple les sels formés avec des acides minéraux ou organiques sur l'amine. Il peut alors s'agir des acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfoniques tels que les acides méthane ou éthane sulfoniques, arylsulfoniques, tels que les acides benzène ou paratoluène sulfoniques et arylcarboxyliques.

Il peut également s'agir des acides chloroacétique ou trichloroacétique. Il s'agit tout particulièrement des sels formés avec l'acide chlorhydrique.

Lorsque R₁ est un groupement alkyle renfermant de 1 à 4 atomes de carbone, il s'agit notamment de méthyle, éthyle, butyle ou propyle, et tout particulièrement d'éthyle.

Par phénylalkyle, on entend de préférence le groupement benzyle.

L'invention a plus particulièrement pour objet le composé de formule (IVd) tel que défini précédemment, ainsi que ses sels d'addition avec les acides.

L'invention a tout particulièrment pour objet le composé de formule (IVd) dans laquelle R₁ est un groupement éthyle, ainsi que ses sels d'addition avec les acides.

La présente invention a d'autre part pour objet un procédé de préparation de composés de formule (IVa), (IVb), (IVc) ou (IVd) tels que décrits plus haut, caractérisé en ce qu'il comporte l'une au moins des étapes suivantes :
a) action d'une arylamine de formule R₃R₄CHNH₂ dans laquelle R₃ est un atome d'hydrogène ou un groupement alkyle renfermant de 1 à 4 atomes de carbone et R₄ est un aryle éventuellement substitué, sur l'alkyloxyfuranone racémique de formule (II) : 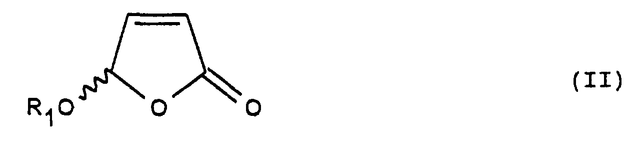 dans laquelle R₁ est un groupement alkyl renfermant de 1 à 4 atomes de carbone, ou phénylalkyle renfermant de 7 à 11 atomes de carbone, et obtention des composés isomères trans (4R,5R) et (4S,5S) respectivement de formules (IIIa) et (IIIb) : 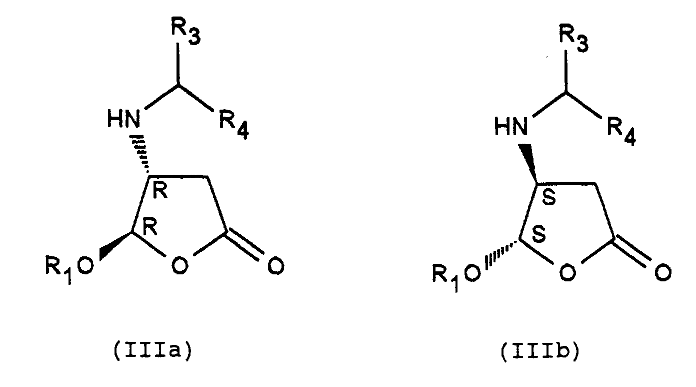 qui ensuite
   - sont séparés par cristallisation en effectuant le cas échéant une ou plusieurs réactions de salification,
   - ou, lorsque R₃ est un atome d'hydrogène, sont dédoublés par action d'un acide optiquemment actif,
b) le cas échéant, réaction d'épimérisation du composé de formule (IIIa) ou (IIIb), salifié ou non salifié, en présence d'un acide, afin d'obtenir les composés isomères cis (4R,5S) ou (4S,5R) de formule (IIIc) ou (IIId), qui sont le cas échéant salifiés, 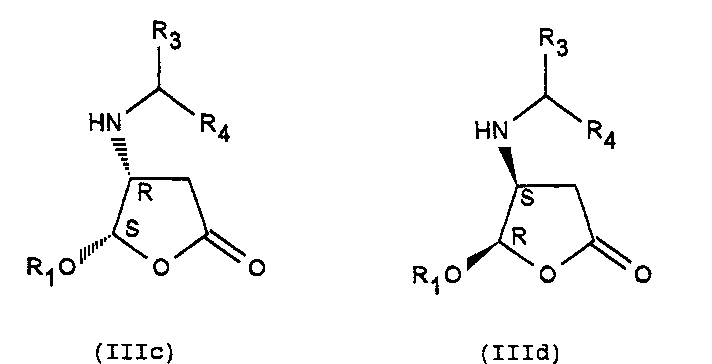
c) réaction d'hydrogénolyse,
   - soit sur l'isomère trans (4R,5R) ou (4S,5S) de formule (IIIa) ou (IIIb), salifié ou non salifié, afin d'obtenir le composé de formule (IVa) ou (IVb) sous la forme de l'isomère trans (4R,5R) ou (4S,5S) tel que défini précédemment, qui est le cas échéant salifié et/ou protégé,
   - soit sur l'isomère cis (4R,5S) ou (4S,5R) de formule (IIIc) ou (IIId), salifié ou non salifié, afin d'obtenir le composé de formule (IVc) ou (IVd) sous la forme de l'isomère cis (4R,5S) ou (4S,5R), tel que défini précédemment, qui est le cas échéant salifié, et/ou protégé.

En particulier la réaction de protection des amines de formule (IVa), (IVb), (IVc) ou (IVd), salifiées ou non salifiées s'effectue par action du chloroformiate de formule Cl-CO-O-CH₂-R₂, R₂ représentant un radical terbutyl, (C₂-C₄)-alkényle, (C₂-C₄)-alkynyle ou phényle, substitué ou non substitué, afin d'obtenir les composés de formule (Ia), (Ib), (Ic) ou (Id), respectivement sous la forme des diastéréoisomères trans (4R,5R), (4S,5S) ou cis (4R,5S), (4S,5R), qui le cas échéant sont salifiés,

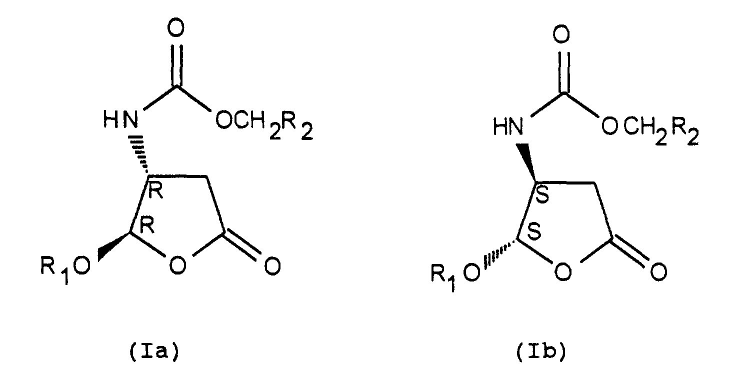

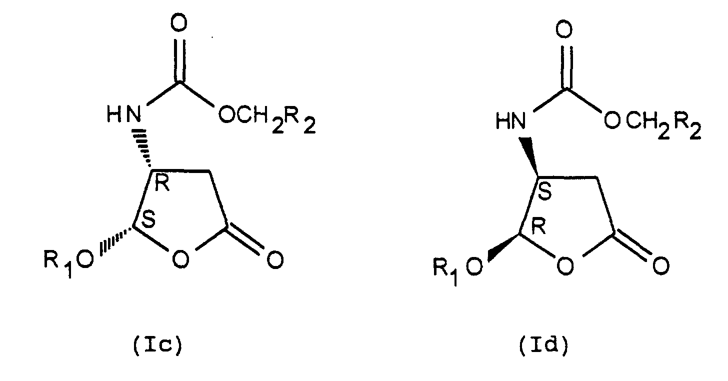

L'action de l'arylamine de formule R₃R₄CHNH₂ sur l'alkyloxyfuranone racémique de formule (II) s'effectue selon les méthodes classiques requises par la réaction de Michael, à savoir notamment dans un solvant aprotique dipolaire tel que le diméthylformamide à température ambiante. On peut opèrer aussi avec la phényléthylamine R ou S ou dans de l'isopropanol aqueux.

La séparation des deux isomères trans (4R,5R) et (4S,5S), à savoir respectivement les composés de formules (IIIa) et (IIIb) par cristallisation s'effectue selon les méthodes connues de l'homme du métier concernant les séparations des isomères. A titre d'exemple préféré la séparation s'effectue par action d'acide trichloroacétique dans un solvant tel que le terbutylméthyléther ou l'isopropanol aqueux. L'isomère trans (4R,5R) (IIIa) est cristallisé sous forme de sel de l'acide trichloroacétique, alors que l'isomère trans (4S,5S) (IIIb) est récupéré sous forme de sel d'acide monochloroacétique par traitement des liqueurs mères en présence d'acide monochloroacétique.

Lorsque R₃ est un atome d'hydrogène (amine non chirale) la séparation (dédoublement) s'effectue alors au moyen d'un acide chiral tel que l'acide tartrique, camphosulfonique, salicylique, dibenzoiltartrique, R⁺ 2,4-hydroxyphénoxypropionique.

La réaction d'épimérisation sur l'un des isomères (4R,5R) ou (4S,5S) de formule (IIIa) ou (IIIb) s'effectue en présence d'un acide de Lewis tel que le chlorure ferrique, le tétrachlorure de titane éventuellement complexé avec du tétrahydrofuranne, le trichlorure de bore, l'éthérate de trifluorure de bore et le tétrachlorure d'étain ou d'un acide organique tel que l'acide méthane sulfonique, l'acide trifluoroacétique et l'acide paratoluènesulfonique. Il s'agit de préférence de tétrachlorure d'étain, en présence d'un solvant peu polaire tel que le dichlorométhane, ou de l'acide méthanesulfonique dans un solvant tel que le toluène.

La réaction d'hydrogénolyse sur les diastéréoisomères cis ou trans de formule (IIIa), (IIIb), (IIIc) ou (IIId), s'effectue selon les méthodes classiques connues de l'homme du métier, par exemple par action d'hydrogène en présence de palladium sur charbon à 10 % dans le tétrahydrofuranne.

La réaction d'acylation avec le chloroformiate s'effectue de préférence en présence d'une base telle que la pyridine, dans un solvant peu polaire tel que le dichlorométhane.

La formation de la base à partir du sel correspondant, c'est-à-dire le retour à l'amine libre, ainsi que les méthodes de salification avec les acides tels que définis précédemment s'effectuent selon les méthodes connues de l'homme du métier.

En ce qui concerne les composés de formule (III) lorsque R₃ est un groupement alkyle renfermant de 1 à 4 atomes de carbone, il s'agit de préférence de méthyle ou d'éthyle et lorsque R₄ est un groupement aryle, il s'agit de préférence de phényle ou naphtyle.

En ce qui concerne les composés de formule (I), lorsque R₂ est un groupement (C₂-C₄)alkényle ou (C₂-C₄) alkynyle, il s'agit de préférence de -CH=CH₂, -C≡CH, -CH=CH₂-CH₃, -C≡C-CH₃.

Les réactions de protection des composés de formule (I) s'effectuent selon les méthodes connues par l'homme du métier et notamment par référence à l'ouvrage de Philip J. Kociensky Protecting Groups Ed. Georg. Thieme Verlog Stuttgart New-York 1994.

La présente invention a plus particulièrement pour objet le procédé tel que défini précédemment, caractérisé en ce que la séparation par cristallisation des composés de formules (IIIa) et (IIIb) s'effectue :
a) par action d'acide trichloroacétique, afin d'obtenir le sel correspondant de formule (IIIa) ou (IIIb),
b) puis par action de l'acide monochloroacétique, sur les liqueurs mères afin d'obtenir le sel correspondant à l'autre diastéréoisomère de formule (IIIa) ou (IIIb).

La présente invention a tout particulièrement pour objet un procédé de préparation tel que décrit précédemment des composés de formule (IVd) ou (Id) tels que définis précédemment, caractérisé en ce qu'il comporte l'une au moins des étapes suivantes :
a) action de la phényléthylamine R sur le composé de formule (II), afin d'obtenir les composés de formules (III'a) et (III'b) suivants : 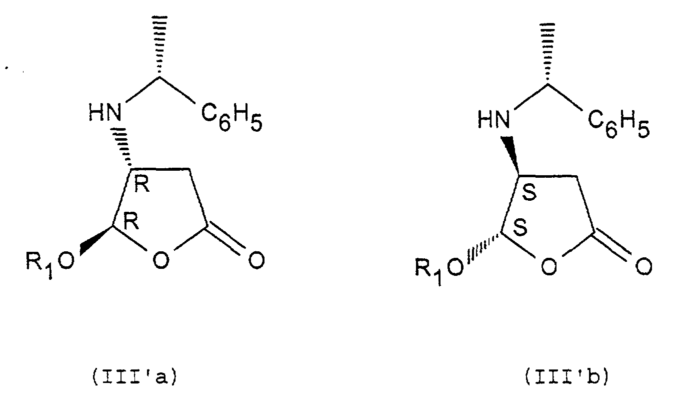
b) séparation des stéréoisomères trans de formules (III'a) et (III'b) par action d'acide trichloroacétique afin d'obtenir le stéréoisomère (III'b) (4S,5S) sous forme de sel d'acide trichloroacétique puis par action d'acide monochloroacétique, afin d'obtenir le stéréoisomère (III'a) (4R,5R) sous forme de sel d'acide monochloroacétique,
c) le cas échéant retour à l'amine libre par action d'une base,
d) réaction d'épimérisation du stéréoisomère (4S,5S) de formule (III'b) en présence d'un acide afin d'obtenir un stéréoisomère cis (4S,5R) de formule (III'd) : 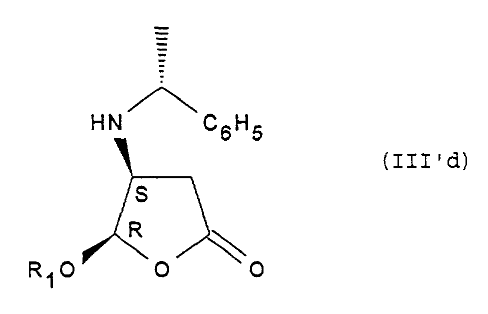
e) le cas échéant cristallisation après salification par action d'un acide tel que l'acide monochloroacétique ou dichloroacétique,
f) le cas échéant retour à l'amine libre par action d'une base,
g) le cas échéant recristallisation après salification notamment sous forme de chlorhydrate,
h) hydrogénolyse du stéréoisomère cis (4S,5R) de formule (III'd), afin d'obtenir le composé de formule (IVd) sous la forme du diastéréoisomère cis (4S,5R)
i) le cas échéant action du chloroformiate d'allyle sur le composé de formule (IVd) afin d'obtenir le composé de formule (Id) sous la forme du diastéréoisomère cis (4S,5R), avec R₁ représentant -CH=CH₂.

La présente invention a également tout particulièrement pour objet un procédé de préparation tel que décrit précédemment des composés de formule (IVd) ou (Id) tels que définis précédemment, caractérisé en ce qu'il comporte l'une au moins des étapes suivantes :
a) action de la phényléthylamine S sur le composé de formule (II), afin d'obtenir les composés de formules (III"a) et (III"b) suivants : 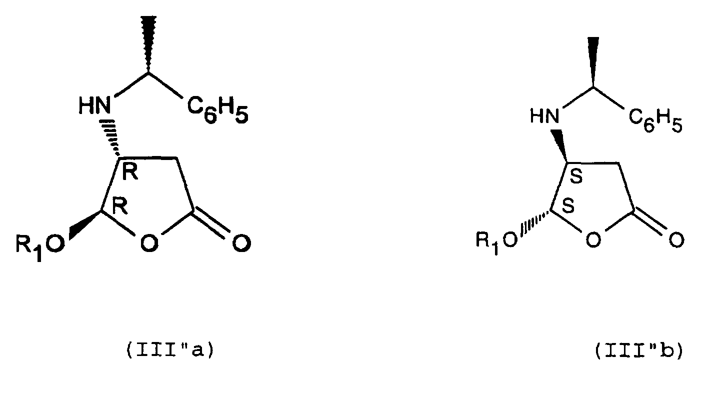
b) dédoublement des stéréoisomères trans de formules (III"a) et (III"b) par action d'acide trichloroacétique afin d'obtenir le stéréoisomère (III"a) (4R,5R) sous forme de sel d'acide trichloroacétique puis par action d'acide monochloroacétique afin d'obtenir le stéréoisomère (III"b) (4S,5S) sous forme de sel d'acide monochloroacétique,
c) le cas échéant retour à l'amine libre par action d'une base,
d) réaction d'épimérisation du stéréoisomère (4S,5S) de formule (III"b) en présence d'un acide afin d'obtenir un diastéréoisomère cis (4S,5R) de formule (III"d) : 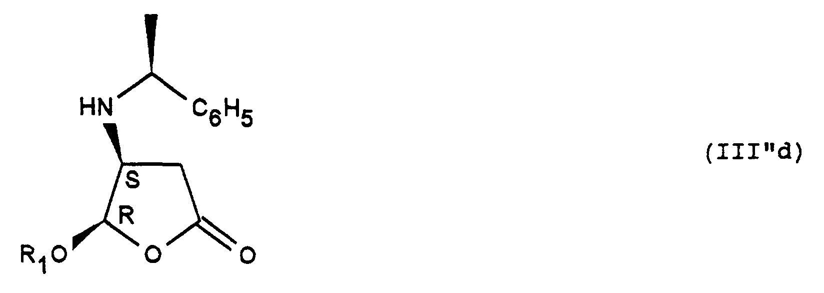
e) le cas échéant cristallisation après salification par action d'un acide tel que l'acide monochloroacétique ou dichloroacétique,
f) le cas échéant retour à l'amine libre par action d'une base,
g) le cas échéant recristallisation après salification notamment sous forme de chlorhydrate,
h) hydrogénolyse du stéréoisomère cis (4S,5R) de formule (III"d), afin d'obtenir le composé de formule (IVd) sous la forme du diastéréoisomère cis (4S,5R)
i) le cas échéant action du chloroformiate d'allyle sur le composé de formule (IVd) afin d'obtenir le composé de formule (Id) sous la forme du diastéréoisomère cis (4S,5R).

L'invention a tout particulièrement pour objet le procédé tel que défini précédemment caractérisé en ce que l'addition de l'aminé sur le composé de formule (II) s'effectue dans le diméthylformamide ou dans l'isopropanol aqueux.

L'invention a tout particulièrement pour objet le procédé tel que défini précédemment caractérisé en ce que la réaction d'épimérisation s'effectue avec le tétrachlorure d'étain ou l'acide méthane sulfonique.

L'invention a tout particulièrement pour objet le procédé tel que défini précédemment caractérisé en ce que R₁ est un radical éthyle.

L'invention a tout particulièrement pour objet le procédé tel que défini précédemment caractérisé en ce que la séparation des stéréoisomères trans avec l'acide trichloroacétique (étape b) s'effectue dans l'isopropanol aqueux.

L'invention a tout particulièrement pour objet le procédé mettant en oeuvre la phényléthylamine (R) caractérisé en ce que la réaction d'épimérisation du stéréoisomère (4S,5S) de formule (III"b) (étape d) s'effectue en présence d'acide méthane sulfonique dans le toluène.

L'invention a tout particulièrement pour objet le procédé mettant en oeuvre la phényléthylamine (R) caractérisé en ce que la cristallisation (étape e) s'effectue par action d'acide dichloroacétique dans le toluène.

L'invention a également pour objet l'utilisation :
soit des composés de formule (IVa), (IVb), (IVc) ou (IVd) tels que définis précédemment ou issus du procédé tel que décrit précédemment,
soit des composés de formule (Ia), (Ib), (Ic) ou (Id) issus du procédé tel que décrit précédemment dans des réactions d'amidification à partir d'acide de formule A-COOH pour obtenir un composé de formule (V) suivant :

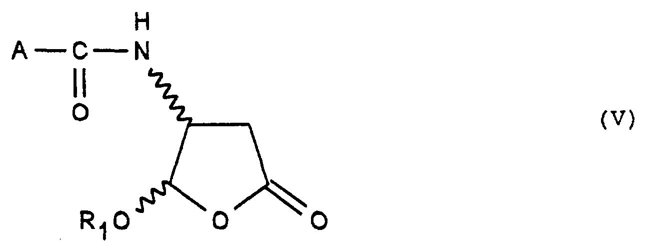

### A représentant un radical organique quelconque.

L'invention a également pour objet l'utilisation : soit des composés de formule (IVb) ou (IVd) tels que définis précédemment ou issus du procédé tel que décrit précédemment, soit des composés de formule (Ib) ou (Id) issus du procédé tel que défini précédemment dans la synthèse des composés de formule (V) ayant une activité inhibiteur de l'enzyme de conversion de l'interleukine.

Ces composés de formule (V) sont notamment décrits dans les demandes de brevets WO9535308, WO9722619, EP0519748 et WO9633209.

L'invention a tout particulièrement pour objet l'utilisation :
soit du composé de formule (IVd) avec R₁ = éthyle tel que défini précédemment ou issu du procédé tel que décrit précédemment,
soit du composé de formule (Id) avec R₁ = éthyle issu du procédé tel que décrit précédemment dans la préparation d'un composé de formule (V) ayant une activité inhibiteur de l'enzyme de conversion de l'interleukine.

L'invention a tout particulièrement pour objet l'utilisation :
soit du composé de formule (IVd) avec R₁ = éthyle tel que défini précédemment ou issu du procédé tel que décrit précédemment,
soit du composé de formule (Id) avec R₁ = éthyle issu du procédé tel que décrit précédemment dans la préparation du composé de formule (V) ayant la structure suivante :

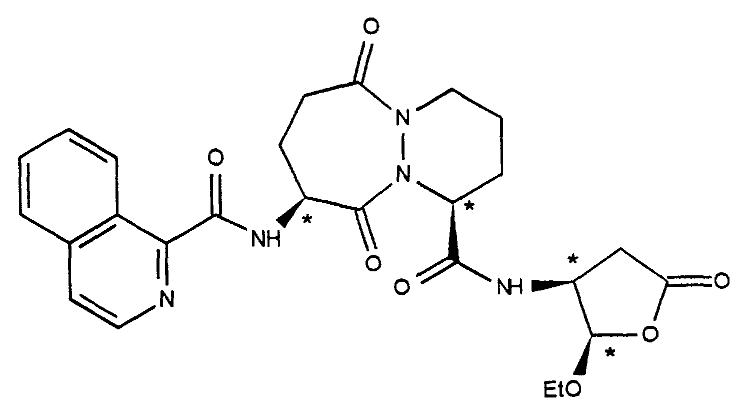

Ce composé étant décrit dans la demande de brevet WO9722619 (Pdt 412e).

L'invention a également pour objet les composés de formules (IIIa), (IIIb), (IIIc), (IIId), ainsi que leurs sels d'addition avec les acides tels que décrits plus haut, à titre de composés intermédiaires nouveaux, à l'exception des composés de formules (III"a) et (III"b) avec R₁ = méthyle.

Les composés de formule (II) sont connus ou aisément accessibles à partir du méthoxyfuranone par action d'APTS (acide paratoluène sulfonique), en présence d'eau puis d'un réactif de formule (R₁O)₃CH en présence d'une catalyse acide.

Les exemples suivants illustrent l'invention sans toutefois la limiter.

### EXEMPLE 1 : (2R-cis) (2-éthoxy-tétrahydro-5-oxo-3-furanyl)-carbamate de 2-propényl

### Stade 1 : Addition de Michael

### 4(R*) dihydro-5-éthoxy-4[(1-phényléthyl)amino]-2(3H)-furanone

On ajoute en 30 minutes entre 23 et 25°C, sous atmosphère inerte, 15 ml de R(+)phényléthylamine à 98 % à 15 g d'éthoxyfuranone racémique dans 75 ml de diméthylformamide, agite 24 heures la solution obtenue puis verse dans le mélange eau/glace. On ajoute de l'éther isopropylique, extrait, lave et sèche, et évapore sous pression réduite jusqu'à obtention de 27 g d'une huile correspondant au mélange des isomères trans 50/50 attendu.

| RMN (CDCl3 ; 250 HZ) | | |
|---|---|---|
| 1,12 ; 1,22 | (t) | CH₂CH₃ |
| 1,37 | (dd) | CH₃ (-NH-CH(CH₃)(Ph)) |
| 2,13 | (dd,J=3,5 et 17,5) ; | |
| 2,35 | (dd, J=3 et 17,5) ; | CH₂ en 3 de la furanone. |
| 2,70 | (dd, J= 7,5 et 17,5) ; | |
| 2,80 | (dd, J = 7 et 17,5) | |
| 3,26 | (m) | CH en 4 de la furanone |
| 3,30 à 3,90 | (m) | CH₂CH₃ |
| 3,82 | (m) | CH (-NH-CH(CH₃)(Ph)) |
| 5,00 | (d,J=1,5) et 5,32 (d,J=1,5) | CH en 5 de la furanone |
| 7,2 à 7,4 | (m) | 5H aromatique |

### Stade 2 : Dédoublement des deux diastéréoisomères trans

### A) obtention du diastéréoisomère trans (4S,5S) trichloroacétate de 4(S)[4α(S*),5β]-dihydro-5-éthoxy-4-[(1-phényléthyl)amino]-2(3H)-furanone

On ajoute en 20 mn une solution de 10,6 g d'acide trichloroacétique (99,5 %) dans 50 ml de méthylterbutyléther à 27 g du mélange de diastéréoisomères obtenus au stade précédent dans 164 ml de méthylterbutyléther. La solution obtenue est agitée 2 heures à 20-25°C puis 2 heures à 0-5°C. On obtient 12 g de l'isomère (4S,5S) sous forme de sel de l'acide trichloroacétique.
[α_{D}] = + 71° (c = 1 % CH₃OH)

| RMN (CDCl3 ; 250 MHz) | | |
|---|---|---|
| 1,15 | (t) | CH₂CH₃ |
| 1,75 | (d,J=7) | CH₃ (-NH-CH(CH₃)(Ph)) |
| 2,78 | (dd,J=8,5 et 18,5) ; | |
| 3,05 | (dd, J=4 et 18,5) ; | CH₂ en 3 de la furanone. |
| 3,43 | (ddd,J=2-4 et 8,5) | CH en 4 de la furanone |
| 3,59 | (dq 1H) 3,79 (dq 1H) | CH₂CH₃ |
| 4,29 | (q,J=7)) | (-NH-CH(CH₃)(Ph)) |
| 5,77 | (d,J=1,5) | CH en 5 de la furanone |
| 7,42 à 7,57 | | 5H aromatique |
| 9,80 | (large) | H mobile |

### B) Obtention du diastéréoisomère trans (4R,5R) chloroacétate de 4(R)[4α(R*),5β]-dihydro-5-éthoxy-4-[(1-phényléthyl)amino]-2(3H)-furanone

Les liqueurs mères du stade précédent sont lavées avec une solution saturée de bicarbonate de sodium puis à l'eau, puis reextraite une fois avec de l'éther isopropylique. Après séchage on évapore sous pression réduite jusqu'à obtention de 15,45 g du produit attendu sous forme d'huile. On ajoute 5 g d'acide monochloroacétique aux 15,45 g de produit dans 130 ml d'isopropanol, et chauffe à 40°C. On observe une dissolution puis une cristallisation, puis agite 1 heure à température ambiante puis 2 heures à 0-5°C. On obtient 11,98 g de l'isomère (4R,5R attendu) sous la forme du sel de l'acide monochloroacétique.

### Stade 3 : obtention du composé (4S,5S) désalifié

### 4(S)[4α(S*),5β]-dihydro-5-éthoxy-4-[(1-phényléthyl)amino]-2(3H)-furanone

On mélange à température ambiante 11,8 g du sel obtenu au stade 2A et 120 ml de dichlorométhane puis on ajoute 100 ml d'une solution saturée de bicarbonate de sodium. Après 10 mn d'agitation, on extrait, lave, sèche et évapore sous pression réduite jusqu'à obtention de 7,1 g du produit désalifié.
[α_{D}] = +114° (c = 1 % CH₃OH)

### Stade 4 : épimérisation : obtention du diastéréoisomère (4S,5R)

### 4(S)[4α(S*),5α]-dihydro-5-éthoxy-4-[(1-phényléthyl)amino]-2(3H)-furanone

On ajoute sous atmosphère inerte en 1 heure à 4 ± 1°C, 28,6 ml de tétrachlorure d'étain 1M dans le dichlorométhane à 6,8 g du composé (4S,5S) désalifié obtenu au stade 3 dans 135 ml de dichlorométhane et agite 40 minutes à cette température. On ajoute ensuite 11 ml d'acide acétique, agite 30 minutes à 5°C, verse dans le mélange eau/glace, lave, ajoute du cyclohexane, amène à pH 7-8 par addition de bicarbonate de sodium, extrait au cyclohexane, sèche et évapore sous pression réduite jusqu'à obtention de 4,39 g du produit attendu sous forme d'une huile correspondant au rapport cis/trans 90/10).
[α_{D}] = -1°5 (c = 1 % CH₃OH)

| RMN (CDCl3 ; 250 MHz) | | |
|---|---|---|
| 1,23 | (t) | CH₂CH₃ |
| 1,40 | (d,J=6,5) | CH₃ (-NH-CH(CH₃)(Ph)) |
| 2,39 | (dd,J=11 et 17) ; | |
| 2,61 | (dd, J=8 et 17) ; | CH₂ en 3 de la furanone. |
| 3,32 | (m) | CH en 4 de la furanone |
| 3,77 | (m) | CH (-NH-CH(CH₃)(Ph)) |
| 4,96 | (d,J=5) | CH en 5 de la furanone |
| 7,20 à 7,40 | (m) | 5H aromatique |

### Stade 5 : formation du sel de l'acide monochloroacétique du diastéréoisomère cis (4S,5R)

### chloroacétate de 4(S)[4α(S*),5α]-dihydro-5-éthoxy-4-[(1-phényléthyl)amino]-2(3H)-furanone

On ajoute 1,82 g d'acide monochloroacétique (96 %), sous atmosphère inerte, à 20-25°C à 5 g du diastéréoisomère cis obtenu au stade précédent dans 50 ml de terbutylméthyléther. On obtient une solution qui cristallise rapidement et on laisse 1 heure 30 à +5°C. On obtient 5,75 g du produit attendu.
F = 106-108°C
[α_{D}] = -11°5 (c = 1 % CH₃OH)

### Stade 6 : hydrogénolyse

### a) désalification (retour à l'amine libre)

### 4(S)[4α(S*),5α]-dihydro-5-éthoxy-4-[(1-phényléthyl)amino]-2(3H)-furanone

On mélange à 0-5°C, sous atmosphère inerte, 5,55 g de sel monochloroacétique obtenu au stade précédent, 60 ml de dichlorométhane et 55 ml de bicarbonate de sodium, agite 10 minutes, lave, extrait, sèche et évapore sous pression réduite jusqu'à obtention de 3,95 g de produit désalifié sous la forme d'une huile.

### b) Formation du chlorhydrate

### chlorhydrate de 4(S)[4α(S*),5α]-dihydro-5-éthoxy-4-[(1-phényléthyl)amino]-2(3H)-furanone

On ajoute, goutte à goutte, à 0-5°C, 6,6 ml d'acide chlorhydrique 2N dans l'éther isopropylique à 3,36 g du produit obtenu précédemment dans 66 ml d'éther isopropylique et maintient 1 heure à cette température. On observe la cristallisation. On obtient 3,77 g de chlorhydrate attendu.

### c) Hydrogénolyse

### chlorhydrate de (4(S)-cis)-4-amino-5-éthoxy-dihydro-2(3H)-furanone

A une suspension de 4 g du chlorhydrate obtenu précédemment dans 60 ml de tétrahydrofuranne on ajoute 1,8 ml d'eau pour obtenir une solution puis 400 mg de palladium sur charbon à 10 %. On agite sous 1,5 bar d'hydrogène pendant 18 heures à 27-28°C. Après filtration et rinçage avec le mélange tétrahydrofuranne/eau on évapore sous pression réduite. On obtient 2,54 g de produit attendu.
[α_{D}] = -96° (c = 1 % CH₃OH)

| | | |
|---|---|---|
| 1,22 | (t) | CH₂CH₃ |
| 2,58 | (dd,J=8 et 17,5) ; | |
| 2,70 | (dd,J=8 et 17,5) ; | CH₂ en 3 de la furanone. |
| 4,14 | (dt, J=5,5 et 8) | CH en 4 de la furanone |
| 3,61 ; 3,90 | (m) | CH₂CH₃ |
| 5,71 | (d,J=5,5) | CH en 5 de la furanone |
| 8,69 | (sl) | 3H mobiles |

### Stade 7 : Formation du carbamate d'allyle

### (2R-cis) (2-éthoxy-tétrahydro-5-oxo-3-furanyl)-carbamate de 2-propenyle

On ajoute à +5°C, sous atmosphère inerte 3 ml de pyridine au mélange constitué de 2,4 g du produit obtenu au stade précédent, 50 ml de dichlorométhane et 1,55 ml de chloroformiate d'allyle, agite une heure à cette température puis ajoute 0,56 ml de chloroformiate d'allyle et 1 ml de pyridine. On agite 6 heures à température ambiante verse dans l'eau, extrait avec du dichlorométhane, lave, sèche, évapore sous pression réduite jusqu'à obtention de 2,66 g de produit brut que l'on recristallise dans de l'éther isopropylique. On obtient 1,95 g de produit pur attendu.
[α_{D}] = -56° (c = 1 % CH₂Cl₂)

| | | |
|---|---|---|
| 1,26 | (t) | CH₂CH₃ |
| 2,47 | (dd,J=10 et 17,5) ; | |
| 2,84 | (dd,J=8,5 et 17,5) ; | CH₂ en 3 de la furanone |
| 3,67 | (dq) et 3,92 (dq) | CH₂CH₃ |
| 4,55 | (m) | CH en 4 de la furanone |
| 4,59 | (dl) | CH₂-CH=CH₂ |
| 5,45 | (d,J=5,5) | CH en 5 de la furanone |
| 5,25 | (dq) et 5,33 (dq) | CH₂-CH=CH₂ |
| 5,30 masqué | | N-H |
| 5,93 | (m) | CH₂-CH=CH₂ |

### EXEMPLE 2 : (2R-cis) (2-éthoxy-tétrahydro-5-oxo-3-furanyl)-carbamate de 2-propenyl

### Stade 1 : Addition de Michael

### 4(S*) dihydro-5-éthoxy-4[(1-phényléthyl)amino]-2(3H)-furanone

On ajoute en 30 minutes entre 23 et 25°C, sous atmosphère inerte, 15 ml de S(-)phényléthylamine à 98 % à 15 g d'éthoxyfuranone racémique dans 75 ml de diméthylformamide, agite 24 heures la solution obtenue puis verse dans le mélange eau/glace. Après extraction avec du cyclohexane, lavage et séchage, on évapore sous pression réduite jusqu'à obtention de 26,6 g d'une huile correspondant au mélange des isomères trans 45/55 attendu.

| RMN (CDCl3 ; 250 MHZ) | | |
|---|---|---|
| 1,12 (t) ; 1,22 | (t) | CH₂CH₃ |
| 1,37 | (dd) | CH₃ (-NH-CH(CH₃)(Ph)) |
| 2,13 | (dd,J=3,5 et 17,5) ; | |
| 2,35 | (dd, J=3 et 127,5) ; | CH₂ en 3 de la furanone |
| 2,70 | (dd, J= 7,5 et 17,5) ; | |
| 2,80 | (dd, J = 7 et 17,5) | |
| 3,26 | (m) | CH en 4 de la furanone |
| 3,30 à 3,90 | (m) | CH₂CH₃ |
| 3,82 | (m) | CH (-NH-CH(CH₃)(Ph)) |
| 5,00 | (d,J=1,5) et 5,32 (d,J=1,5) | CH en 5 de la furanone |
| 7,2 à 7.4 | (m) | 5H aromatique |

### Stade 2 : Dédoublement des deux diastéréoisomères trans

### a) obtention du diastéréoisomère trans (4R,5R) trichloroacétate de 4(R)[4α(S*),5β]-dihydro-5-éthoxy-4-[(1-phényléthyl)amino]-2(3H)-furanone

On ajoute en 30 mn une solution de 10,24 g d'acide trichloroacétique (99,5 %) dans 50 ml de terbutyléther à 26 g du mélange du stade précédent dans 155 ml de terbutyléther. La solution obtenue est agitée 2 heures à 20-25°C puis 2 heures à 5°C. On obtient 12,63 g de l'isomère (4R,5R) sous forme de sel de l'acide trichloroacétique
[α_{D}] = -72°5 (c = 1 % CH₃OH)

| RMN (CDCl3 ; 250 MHZ) | | |
|---|---|---|
| 1,15 | (t) | CH₂CH₃ |
| 1,75 | (d,J=7) | CH₃ (-NH-CH(CH₃)(Ph)) |
| 2,78 | (dd,J=8,5 et 18,5) ; | |
| 3,05 | (dd, J=4 et 18,5) ; | CH₂ en 3 de la furanone |
| 3,43 | (ddd,J=2-4 et 8,5) | CH en 4 de la furanone |
| 3,59 | (dq 1H) 3,79 (dq 1H) | CH₂CH₃ |
| 4,29 | (q,J=7) | (-NH-CH(CH₃)(Ph)) |
| 5,77 | (d,J=1,5) | CH en 5 de la furanone |
| 7,42 à 7,57 | | 5H aromatique |
| 9,80 | (m large) | H mobile |

### b) obtention du diastéréoisomère trans (4S,5S)

### chloroacétate de 4(S)[4α(R*),5β]-dihydro-5-éthoxy-4-[(1-phényléthyl)amino]-2(3H)-furanone

Les liqueurs mères du stade précédent sont lavées avec une solution saturée de bicarbonate de sodium, puis reextraites avec du terbutyléther. Après séchage on évapore sous pression réduite jusqu'à obtention de 17,43 g du produit attendu sous forme d'huile. On ajoute 130 ml d'isopropanol puis 5 g d'acide monochloroacétique et chauffe à 40°C. On observe une dissolution puis une cristallisation, puis agite 1 heure à température ambiante puis 2 heures à 0-5°C. On obtient 12,48 g de l'isomère (4S,5S attendu) sous la forme du sel de l'acide monochloroacétique.
[α_{D}] = +1° (c = 1 % CH₃OH)

| RMN (CDCl3 ; 250 MHZ) | | |
|---|---|---|
| 1,12 | (t, J=7,5) | CH₂CH₃ |
| 1,26 | (d,J=6,5) | CH₃ (-NH-CH(CH₃)(Ph)) |
| 2,18 | (dd,J=2,5 et 17,5) ; | |
| 2,66 | (dd, J=7,5 et 17,5) ; | CH₂ en 3 de la furanone |
| 2,95 | (ddd,J=1-2,5 et 7,5) | CH en 4 de la furanone |
| 3,66 | (m) | CH₂CH₃ |
| 3,87 | (q,J=6,5) | CH (-NH-CH(CH₃)(Ph)) |
| 5,42 | (d,J=1) | CH en 5 de la furanone |
| 7,24 | (m) 1H, 7,33 (m) 4H | H aromatique |
| 4,26 | (s) | X-CH₂ |

### Stade 3 : obtention du composé (4S,5S) désalifié

### 4(S)[4α(R*),5β]-dihydro-5-éthoxy-4-[(1-phényléthyl)amino]-2(3H)-furanone

On mélange à 0-5°C 12,35 g du sel obtenu au stade 2B et 130 ml de dichlorométhane puis on ajoute 100 ml d'une solution saturée de bicarbonate de sodium. Après 10 mn d'agitation, on extrait, lave, sèche et évapore sous pression réduite jusqu'à obtention de 8,9 g du produit désalifié.
[α_{D}] = -6,6° (c = 1 % CH₃OH)

### Stade 4 : épimérisation : obtention du diastéréoisomère (4S,5R)

### 4(S)[4α(R*),5α]-dihydro-5-éthoxy-4-[(1-phényléthyl)amino]-2(3H)-furanone

On ajoute sous atmosphère inerte en 45 minutes à 0-5°C, 37 ml de tétrachlorure d'étain 1M dans le dichlorométhane à 8,8 g du composé (4S,5S) désalifié obtenu au stade 3 dans 175 ml de dichlorométhane et agite 1 heure à cette température. On ajoute ensuite 14,1 ml d'acide acétique, agite 1 heure à 0-5°C, verse dans le mélange eau/glace, lave, ajoute du cyclohexane, amène à pH 7-8 par addition de bicarbonate de sodium, extrait au cyclohexane, sèche et évapore sous pression réduite jusqu'à obtention de 3,96 g du produit attendu sous forme d'une huile correspondant au rapport cis/trans 90/10).

| RMN (CDCl3 ; 250 MHZ) | | |
|---|---|---|
| 1,29 | (t) | CH₂CH₃ |
| 1,35 | (d) | CH₃ (-NH-CH(CH₃)(Ph)) |
| 2,28 | (dd,J=11,5 et 17) ; | |
| 2,43 | (dd, J=8 et 17) ; | CH₂ en 3 de la furanone. |
| 3,36 | (ddd, J=4,5/8/11,5) | CH en 4 de la furanone |
| 3,67 | (dq) ; 3,92 (dq) | CH₂CH₃ |
| 3,81 | (q) | CH (-NH-CH(CH₃)(Ph)) |
| 5,79 | (d,J=4,5) | CH en 5 de la furanone |
| 7,20 à 7,40 | (m) | 5H aromatique |

### Stade 5 : formation du sel de l'acide trichloroacétique du diastéréoisomère cis (4S,5R)

### Trichloroacétate de 4(S)[4α(R*),5α]-dihydro-5-éthoxy-4-[(1-phényléthyl)amino]-2(3H)-furanone

On ajoute 2,34 g d'acide trichloroacétique (99 %), sous atmosphère inerte, à 20-25°C à 3,8 g du diastéréoisomère cis obtenu au stade précédent dans 40 ml de terbutylméthyléther. On laisse 1 heure à 0-5°C et on obtient 5,58 g du produit attendu.
[α_{D}] = -49° (c = 0,9 % CH₃OH)

### Stade 6 : hydrogénolyse

### a) désalification (retour à l'amine libre)

### 4(S)[4α(R*),5α]-dihydro-5-éthoxy-4-[(1-phényléthyl)amino]-2(3H)-furanone

On mélange à 0-5°C, sous atmosphère inerte, 5,41 g de sel trichloroacétique obtenu au stade précédent, 50 ml de cyclohexane et 50 ml de solution saturée de bicarbonate de sodium, agite jusqu'à dissolution, lave, extrait, sèche et évapore sous pression réduite jusqu'à obtention de 3,18 g de produit désalifié sous la forme d'une huile.
[α_{D}] = -93° (c = 0,62 % CH₃OH)

On repurifie ce produit en mélangeant pendant 10 minutes 2,85 g de ce produit avec 30 ml de cyclohexane et 2,8 g de silice. Après traitement on recueille 2,3 g d'huile incolore.

### b) formation du chlorhydrate

### chlorhydrate de 4(S)[4α(R*),5α]-dihydro-5-éthoxy-4-[(1-phényléthyl)amino]-2(3H)-furanone

On ajoute, goutte à goutte, à 0-5°C, 4 ml d'acide chlorhydrique 2N dans l'éther isopropylique à 2 g du produit obtenu précédemment dans 40 ml d'éther isopropylique et maintient 15 minutes à cette température. On évapore sous pression réduite jusqu'à obtention de 2,29 g de chlorhydrate attendu.

### c) hydrogénolyse

### chlorhydrate de (4(S)-cis)-4-amino-5-éthoxy-dihydro-2(3H)-furanone

A une suspension de 2,29 g du chlorhydrate obtenu précédemment dans 40 ml de tétrahydrofuranne on ajoute 1 ml d'eau puis 300 mg de palladium sur charbon à 10 %. On agite sous 1,5 bar d'hydrogène pendant 5 heures. Après filtration et rinçage avec le mélange tétrahydrofuranne/eau on évapore sous pression réduite à 45°C. On obtient 1,40 g de produit attendu.
[α_{D}] = -87°4 (c=1 % CH₃OH)

| | | |
|---|---|---|
| 1,22 | (t) | CH₂CH₃ |
| 2,58 | (dd,J=8 et 17,5) ; | |
| 2,70 | (dd,J=8 et 17,5) ; | CH₂ en 3 de la furanone |
| 4,14 | (dt, J=5,5 et 8) | CH en 4 de la furanone |
| 3,61 à 3,9 | (m) | CH₂CH₃ |
| 5,71 | (d,J=5,5) | CH en 5 de la furanone |
| 8,69 | (sl) | 3H mobiles |

### Stade 7 : Formation du carbamate d'allyle

### (2R-cis) (2-éthoxy-tétrahydro-5-oxo-3-furanyl)-carbamate de 2-propenyl

On ajoute à +5°C, sous atmosphère inerte 1 ml de chloroformiate d'allyle à 99 % puis 2 ml de pyridine à 1,3 g du produit obtenu au stade précédent dans 50 ml de dichlorométhane, agite une heure 40 à cette température puis ajoute 0,3 ml de chloroformiate d'allyle et 0,6 ml de pyridine. On agite 16 heures à température ambiante verse dans l'eau, extrait avec du dichlorométhane, lave, sèche, évapore sous pression réduite jusqu'à obtention de 1,62 g de produit brut que l'on recristallise dans de l'éther isopropylique. On obtient 1,34 g de produit attendu.
[α_{D}] = -52,4° (c=1% CH₂Cl₂)

| | | |
|---|---|---|
| 1,26 | (t) | CH₂CH₃ |
| 2,47 | (dd,J=10 et 17,5) ; | |
| 2,84 | (dd,J=8,5 et 17,5) ; | CH₂ en 3 de la furanone |
| 3,67 | (dq) et 3,92 (dq) | CH₂CH₃ |
| 4,55 | (m) | CH en 4 de la furanone |
| 4,59 | (dl) | CH₂-CH=CH₂ |
| 5,45 | (d,J=5,5) | CH en 5 de la furanone |
| 5,25 | (dq) et (5,33 (dq) | CH₂-CH=CH₂ |
| 5,30 masqué | | N-H |
| 5,93 | (m) | CH₂-CH=CH₂ |

### EXEMPLE 3 : dichloroacétate de 4(S)[4α(S*),5α]-dihydro-5-éthoxy-4-[(1-phényléthyl) amino]-2(3H)-furanone

### Stade 1 : Addition de Michael

### 4(R*) dihydro-5-éthoxy-4[(1-phényléthyl)amino]-2(3H)-furanone

On ajoute en 1 h 30 environ sous agitation et atmosphère d'azote en maintenant la température à 0±2°C, 20 ml de R(+)-1-phényléthylamine (19,06 g) à une solution de 20 g d'éthoxyfuranone racémique dans 156,8 ml d'isopropanol et 3,2 ml d'eau et agite 24 heures à cette température.

### Stade 2 : Dédoublement des deux diastéréoisomères trans : obtention du diastéréoisomère trans (4S,5S)

### trichloroacétate de 4(S)[4α(S*),5β]-dihydro-5-éthoxy-4-[(1-phényléthyl)amino]-2(3H)-furanone

On ajoute à la solution précédente (le produit n'est pas isolé) la solution constituée de 25,55 g d'acide trichloroacétique dans 39,2 ml d'isopropanol et 0,8 ml d'eau déminéralisée, laisse la température évoluer à 20-22°C, et observe la cristallisation du sel après la fin de l'introduction. Cette suspension est maintenue pendant 24 heures à 20±2°C, puis le produit est essoré et lavé avec de l'isopropanol à 2 % d'eau. On obtient 18 g du produit attendu.

| | | |
|---|---|---|
| 1,15 | (t) | O-CH₂-CH₃ |
| 1,75 | (d,J=7) | Ph-CH(CH₃)-N |
| 2,78 | (dd,J=8,5 et 18,5) ; | CH₂ en 3 |
| 3,05 | (dd,J=4 et 18,5) ; | |
| 3,43 | (ddd,J=2,4 et 8,5) ; | H₄ |
| 3,59 | (dq) 1H, 3,79 (dq) 1H ; | O-CH₂-CH₃ |
| 4,29 | (q,J=7) ; | Ph-CH(CH₃)-N |
| 5,77 | (d,J=1,5) ; | H₅ |
| 7,42 à 7,57 | (5H) ; | H aromatiques |
| 9,80 | large ; | H mobile. |

### Stade 3 : épimérisation : obtention du diastéréoisomère (4S,5R)

### 4(S)[4α(S*),5α]-dihydro-5-éthoxy-4-[(1-phényléthyl)amino]-2(3H)-furanone

On ajoute sous azote, en maintenant la température à 20±2°C, 45 ml d'acide méthane sulfonique dans une suspension de 50 g du trichloroacétate (4S,5S) obtenu au stade précédent dans 150 ml de toluène, maintient pendant 2 heures à 20±2°C et après avoir abaissé la température à 0+5°C, ajoute 111 ml de triéthylamine en 1 h 30. Après une série de lavage, extraction et séchage, on obtient 400 ml d'une solution contenant un mélange 85/15 d'isomère cis/trans.

### Stade 4 : formation du sel de l'acide dichloroacétique du diastéréoisomère cis (4S,5R)

### dichloroacétate de 4(S)[4α(S*),5α]-dihydro-5-éthoxy-4-[(1-phényléthyl)amino]-2(3H)-furanone

On ajoute 10 ml d'acide dichloroacétique à 400 ml de la solution précédente, concentre à 6 volumes, observe la cristallisation, et maintient sous agitation à 20±2°C pendant 2 heures sous azote. Après lavage avec du toluène, on obtient 32,8 g de produit attendu.

| | | |
|---|---|---|
| 1,21 | (t) | O-CH₂-CH₃ |
| 1,72 | (d,J=6,5) | Ph-CH(CH₃)-N |
| 2,77 | (dd,J=8,5 et 17) ; | CH₂ en 3 |
| 2,97 | (dd,J=11 et 17) ; | |
| 3,76 | (m) ; | H₄ |
| 3,34 | (dq), 3,66 (dq) ; | O-CH₂-CH₃ |
| 4,27 | (q,J=6,5) ; | Ph-CH(CH₃)-N |
| 4,88 | (d,J=5) ; | H₅ |
| 5,95 | (s) ; | CHCl₂ |
| 7,42 | (m) 3H, 7,51 (m) 2H ; | H aromatiques. |
| 9,79 | (sl) | 2H mobiles. |

## Revendications

1. Composés de formule (IVa), (IVb), (IVc) ou (IVd) suivants : 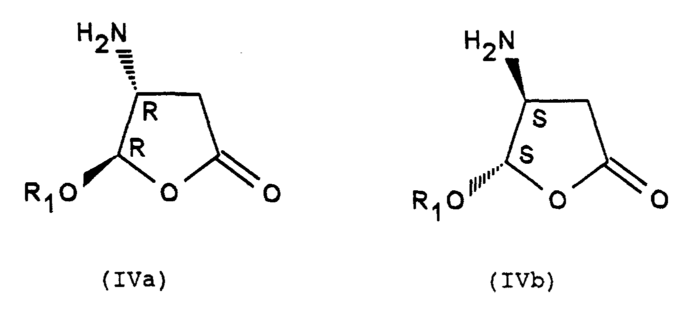 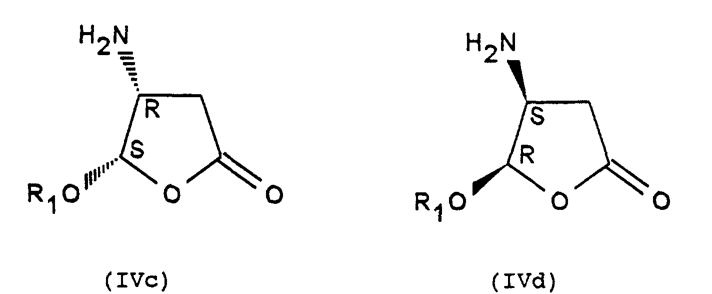 dans lesquelles R₁ est un groupement alkyle renfermant de 1 à 4 atomes de carbone ou un groupement phénylalkyle renfermant de 7 à 11 atomes de carbone, ainsi que leurs sels d'addition avec les acides.

2. Composé de formule (IVd) telle que définie à la revendication 1, ainsi que ses sels d'addition avec les acides.

3. Composé de formule (IVd) selon la revendication 2 dans laquelle R₁ est un groupement éthyle, ainsi que ses sels d'addition avec les acides.

4. Procédé de préparation des composés de formule (IVa), (IVb), (IVc) ou (IVd) telles que définies à la revendication 1, **caractérisé en ce qu'**il comporte l'une au moins des étapes suivantes :
a) action d'une arylamine de formule R₃R₄CHNH₂ dans laquelle R₃ est un atome d'hydrogène ou un groupement alkyle renfermant de 1 à 4 atomes de carbone et R₄ est un aryle éventuellement substitué, sur l'alkyloxyfuranone racémique de formule (II) : 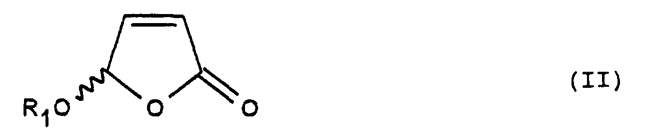 dans laquelle R₁ est un groupement alkyl renfermant de 1 à 4 atomes de carbone, ou phénylalkyle renfermant de 7 à 11 atomes de carbone,
et obtention des composés isomères trans (4R,5R) et (4S,5S) respectivement de formules (IIIa) et (IIIb) 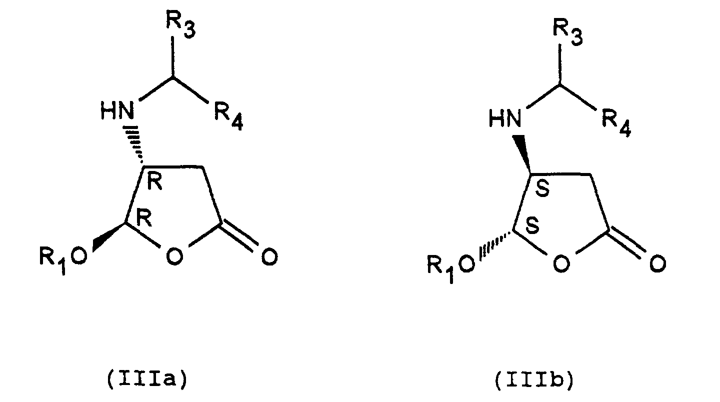 qui ensuite
- sont séparés par cristallisation en effectuant, le cas échéant, une ou plusieurs réactions de salification,
- ou, lorsque R₃ est un atome d'hydrogène, sont dédoublés par action d'un acide optiquement actif,
b) le cas échéant, réaction d'épimérisation du composé de formule (IIIa) ou (IIIb), salifié ou non salifié, en présence d'un acide, afin d'obtenir des composés isomères cis (4R,5S) ou (4S,5R) de formule (IIIc) ou (IIId), qui sont le cas échéant salifiés, 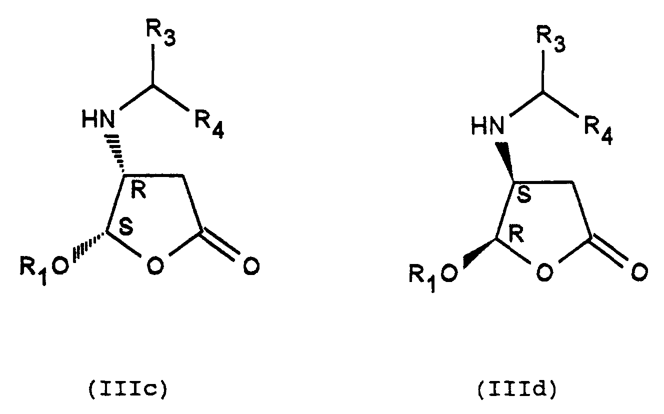
c) réaction d'hydrogénolyse,
- soit sur l'isomère trans (4R,5R) ou (4S,5S) de formule (IIIa) ou (IIIb), salifié ou non salifié, afin d'obtenir le composé de formule (IVa) ou (IVb) sous la forme de l'isomère trans (4R,5R) ou (4S,5S), qui est le cas échéant salifié et/ou protégé,
- soit sur l'isomère cis (4R,5S) ou (4S,5R) de formule (IIIc) ou (IIId), salifié ou non salifié, afin d'obtenir le composé de formule (IVc) ou (IVd) sous la forme de l'isomère cis (4R,5S) ou (4S,5R), qui est le cas échéant salifié et/ou protégé.

5. Procédé selon la revendication 4, **caractérisé en ce que** la réaction de protection des amines de formule (IVa), (IVb), (IVc) ou (IVd) salifiés ou non salifiés s'effectue par action du chloroformiate de formule Cl-CO-O-CH₂-R₂, R₂ représentant un radical terbutyl, (C₂-C₄)-alkényle,(C₂-C₄)- alkynyle ou phényle, substitué ou non substitué, afin d'obtenir les composés de formule (Ia), (Ib), (Ic) ou (Id), respectivement sous la forme des diastéréoisomères trans (4R,5R), (4S,5S) ou cis (4R,5S), (4S,5R), qui le cas échéant sont salifiés,

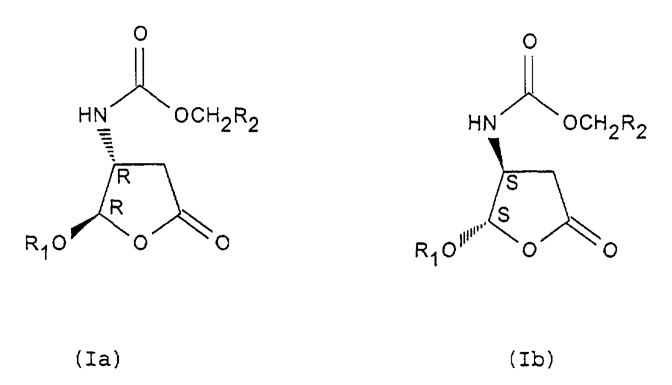

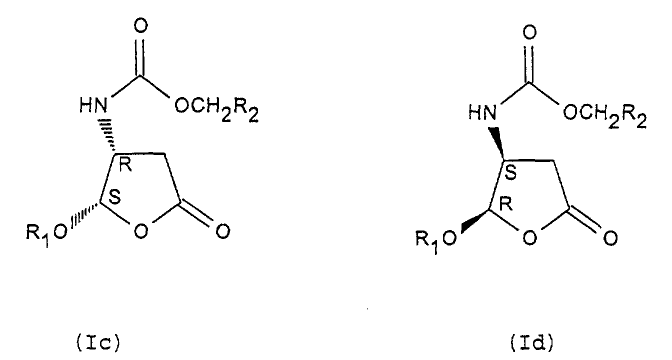

6. Procédé selon la revendication 4 **caractérisé en ce que** la séparation par cristallisation des composés de formules (IIIa) et (IIIb) telles que définies à la revendication 4, s'effectue :
a) par action d'acide trichloroacétique, afin d'obtenir le sel correspondant de formule (IIIa) ou (IIIb),
b) puis par action d'acide monochloroacétique sur les liqueurs mères afin d'obtenir le sel correspondant à l'autre diastéréoisomère de formule (IIIa) ou (IIIb).

7. Procédé de préparation selon la revendication 4, des composés de formule (IVd) tels que définis à la revendication 2 ou des composés de formule (Id) tels que définis à la revendication 5, **caractérisé en ce qu'**il comporte l'une au moins des étapes suivantes :
a) action de la phényléthylamine R sur le composé de formule (II), afin d'obtenir les composés de formules (III'a) et (III'b) suivants 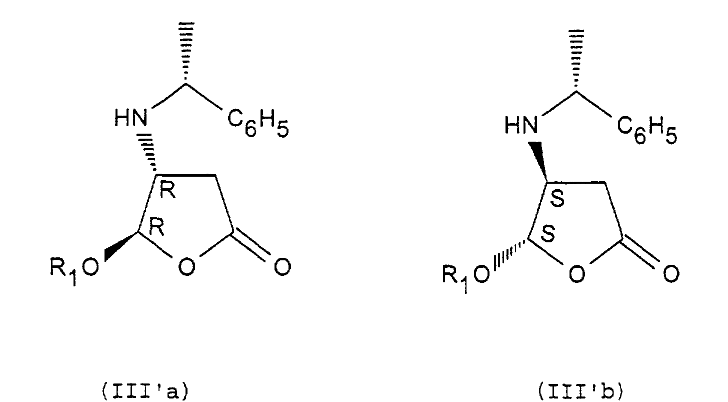
b) dédoublement des stéréoisomères trans de formules (III'a) et (III'b) par action d'acide trichloroacétique afin d'obtenir le stéréoisomère (III'b) (4S,5S) sous forme de sel d'acide trichloroacétique puis par action d'acide monochloroacétique, afin d'obtenir le stéréoisomère (III'a)(4R,5R) sous forme de sel d'acide monochloroacétique,
c) le cas échéant retour à l'amine libre par action d'une base,
d) réaction d'épimérisation du stéréoisomère (4S,5S) de formule (III'b) en présence d'un acide afin d'obtenir un stéréoisomère cis (4S,5R) de formule (III'd) : 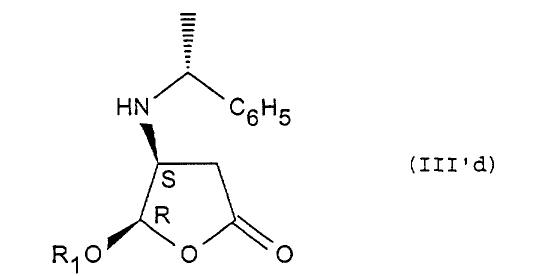
e) le cas échéant cristallisation après salification par action d'acide monochloroacétique ou dichloroacétique,
f) le cas échéant retour à l'amine libre par action d'une base,
g) le cas échéant recristallisation après salification notamment sous forme de chlorhydrate,
h) hydrogénolyse du stéréoisomère cis (4S,5R) de formule (III'd), afin d'obtenir le composé de formule (IVd) sous la forme du diastéréoisomère cis (4S,5R),
i) le cas échéant action du chloroformiate d'allyle sur le composé de formule (IVd) afin d'obtenir le composé de formule (Id) sous la forme du diastéréoisomère cis (4S,5R) avec R2 représentant -CH=CH₂.

8. Procédé de préparation selon la revendication 4 des composés de formule (IVd) tels que définis à la revendication 2 ou des composés de formule (Id), tels que définis à la revendication 5, **caractérisé en ce qu'**il comporte l'une au moins des étapes suivantes :
a) action de la phényléthylamine S sur le composé de formule (II), afin d'obtenir les composés de formules (III"a) et (III"b) suivants : 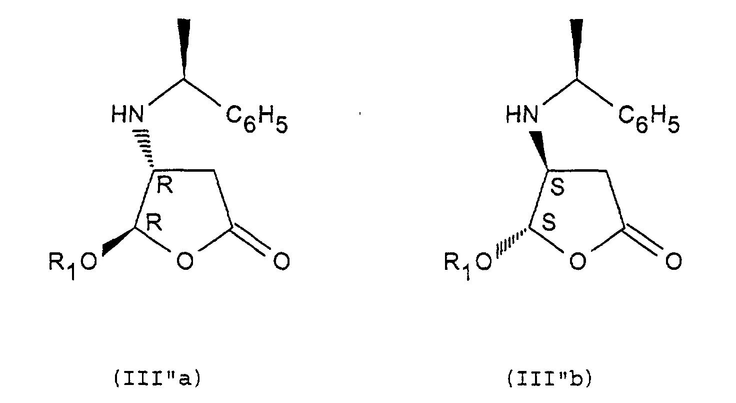
b) dédoublement des stéréoisomères trans de formules (III"a) et (III"b) par action d'acide trichloroacétique afin d'obtenir le stéréoisomère (III"a) (4R,5R) sous forme de sel d'acide trichloroacétique puis par action d'acide monochloroacétique, afin d'obtenir le stéréoisomère (III"b) (4S,5S) sous forme de sel d'acide monochloroacétique,
c) le cas échéant désalification par action d'une base,
d) réaction d'épimérisation du stéréoisomère (4S,5S) de formule (III"b) en présence d'un acide afin d'obtenir le stéréoisomère cis (4S,5R) de formule (III"d) : 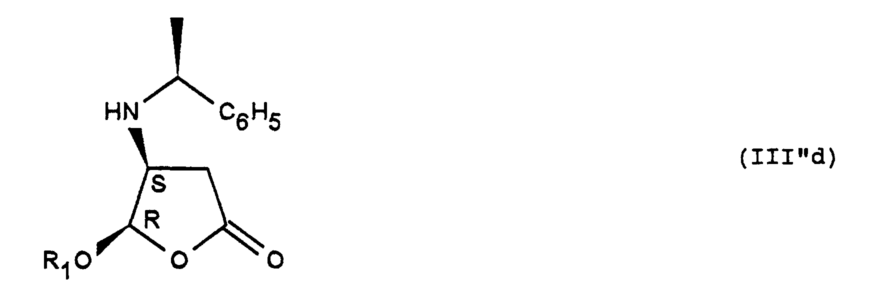
e) le cas échéant cristallisation après salification par action d'un acide tel que l'acide monochloroacétique ou dichloroacétique,
f) le cas échéant retour à l'amine libre par action d'une base,
g) le cas échéant recristallisation après salification notamment sous forme de chlorhydrate,
h) hydrogénolyse du stéréoisomère cis (4S,5R) de formule (III"d), afin d'obtenir le composé de formule (IVd) sous la forme du diastéréoisomère cis (4S,5R)
i) le cas échéant action du chloroformiate d'allyle sur le composé de formule (IVd) afin d'obtenir le composé de formule (Id) sous la forme du diastéréoisomère cis (4S,5R), R₂ représentant un radical -CH=CH₂.

9. Procédé tel que défini à l'une quelconque des revendications 4 à 8 **caractérisé en ce que** l'addition de l'amine sur le composé de formule (II) tel que défini à la revendication 4, s'effectue dans le diméthylformamide ou l'isopropanol aqueux.

10. Procédé tel que défini à l'une quelconque des revendications 4 à 8 **caractérisé en ce que** la réaction d'épimérisation s'effectue avec le tétrachlorure d'étain ou l'acide méthane sulfonique.

11. Procédé tel que défini à l'une quelconque des revendications 4 à 8 **caractérisé en ce que** R₁ est un radical éthyle.

12. Procédé tel que défini à l'une quelconque des revendications 8 à 11, **caractérisé en ce que** le dédoublement des stéréoisomères trans avec l'acide trichloroacétique (étape b) s'effectue dans l'isopropanol aqueux.

13. Procédé tel que défini à l'une quelconque des revendications 8 à 11, **caractérisé en ce que** la réaction d'épimérisation du stéréoisomère (4S,5S) de formule (III"b, étape d) s'effectue en présence d'acide méthane sulfonique dans le toluène.

14. Procédé tel que défini à l'une quelconque des revendications 8 à 11, **caractérisé en ce que** la cristallisation (étape e) s'effectue par action d'acide dichloroacétique dans le toluène.

15. Utilisation :
soit des composés de formule (IVa), (IVb), (IVc) ou (IVd) tels que définis à la revendication 1 ou issus du procédé tel que défini à la revendication 4,
soit des composés de formule (Ia), (Ib), (Ic) ou (Id) issus du procédé tel que défini à la revendication 5 dans des réactions d'amidification à partir d'acide de formule A-COOH pour obtenir un composé de formule (V) suivant : 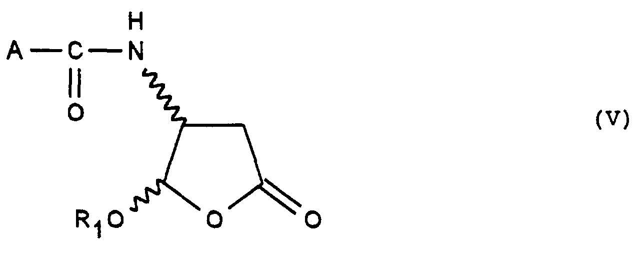 A représentant un radical organique quelconque.

16. Utilisation selon la revendication 15
soit des composés de formule (IVb) ou (IVd) tels que définis à la revendication 1 ou issus du procédé tel que défini à la revendication 4,
soit des composés de formule (Ib) ou (Id) issus du procédé tel que défini à la revendication 5 dans la synthèse des composés de formule (V) ayant une activité inhibiteur de l'enzyme de conversion de l'interleukine.

17. Utilisation selon la revendication 15
soit du composé de formule (IVd) avec R₁ = éthyle, tel que défini à la revendication 3 ou issu du procédé tel que défini à la revendication 7 ou 8,
soit du composé de formule (Id) avec R₁ = éthyle, issu du procédé tel que défini à la revendication 5 dans la préparation d'un composé de formule (V) ayant une activité inhibiteur de l'enzyme de conversion de l'interleukine.

18. Utilisation selon la revendication 17 des composés de formule (IVd) ou (Id) tels que définis à la revendication 17, dans la préparation du composé de formule (V) ayant la structure suivante :

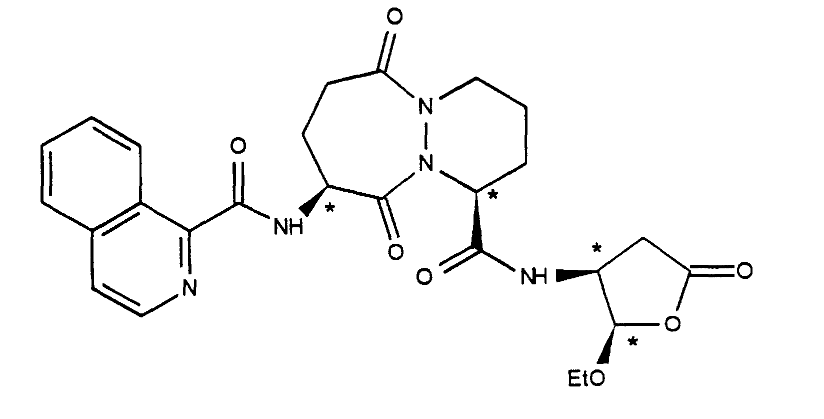

19. Composés de formules (IIIa), (IIIb), (IIIc), (IIId), à titre de composés intermédiaires nouveaux, ainsi que leurs sels d'addition avec les acides, à l'exception des composés de formules (III"a) et (III"b) tels que définis à la revendication 8 avec R₁ = méthyle.

## Claims

1. The following compounds of formula (IVa), (IVb), (IVc) or (IVd): 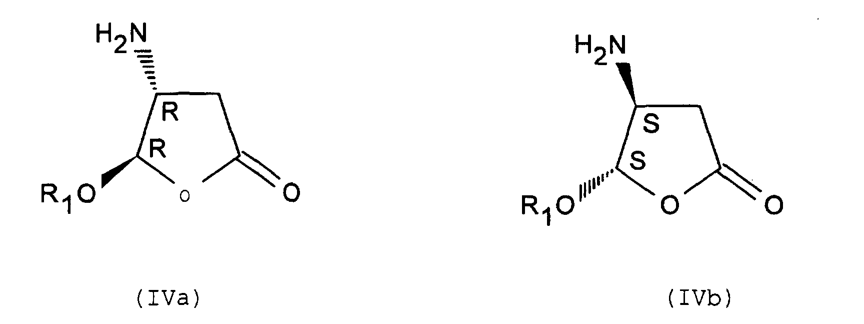 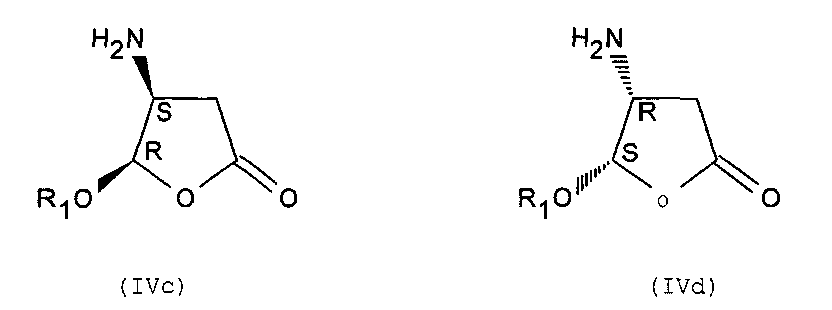 in which R₁ is an alkyl group containing 1 to 4 carbon atoms or a phenylalkyl group containing 7 to 11 carbon atoms, as well as their addition salts with acids.

2. Compound of formula (IVd) as defined in claim 1, as well as its addition salts with acids.

3. Compound of formula (IVd) according to claim 2, in which R₁ is an ethyl group, as well as its addition salts with acids.

4. Process for the preparation of the compounds of formula (IVa), (IVb), (IVc) or (IVd) as defined in claim 1, **characterized in that** it includes at least one of the following stages:
a) the action of an arylamine of formula R₃R₄CHNH₂ in which R₃ is a hydrogen atom or an alkyl group containing 1 to 4 carbon atoms and R₄ is an optionally substituted aryl, on the racemic alkyloxyfuranone of formula (II): 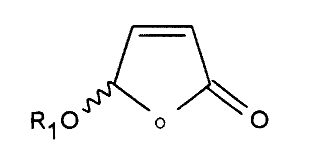 in which R₁ is an alkyl group containing 1 to 4 carbon atoms, or phenylalkyl containing 7 to 11 carbon atoms, and obtaining trans (4R,5R) and (4S,5S) isomer compounds of formulae (IIIa) and (IIIb) respectively 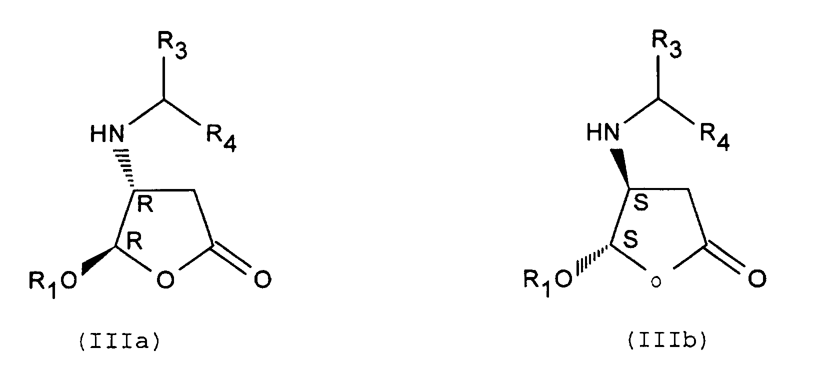 which then
- are separated by crystallization by carrying out, if appropriate, one or more salification reactions,
- or, when R₃ is a hydrogen atom, are resolved by the action of an optically active acid,
b) if appropriate, epimerization reaction of the compound of formula (IIIa) or (IIIb), salified or non salified, in the presence of an acid, in order to obtain the cis (4R,5S) or (4S,5R) isomer compounds of formula (IIIc) or (IIId), which if appropriate are salified, 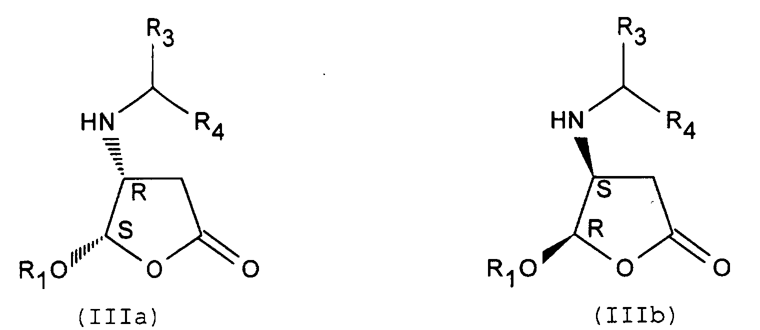
c) hydrogenolysis reaction,
- either on the trans (4R,5R) or (4S,5S) isomer of formula (IIIa) or (IIIb), salified or non salified, in order to obtain the compound of formula (IVa) or (IVb) in the form of the trans (4R,5R) or (4S,5S) isomer, which if appropriate is salified and/or protected,
- or on the cis (4R, 5S) or (4S, 5R) isomer of formula (IIIc) or (IIId), salified or non salified, in order to obtain the compound of formula (IVc) or (IVd) in the form of the cis (4R,5S) or (4S,5R) isomer, which if appropriate is salified and/or protected.

5. Process according to claim 4, **characterized in that** the protection reaction of the amines of formula (IVa), (IVb), (IVc) or (IVd), salified or non salified, is carried out by the action of the chloroformate of formula Cl-CO-O-CH₂-R₂, R₂ representing a substituted or non substituted terbutyl, (C₂-C₄)-alkenyl, (C₂-C₄)- alkynyl or phenyl radical, in order to obtain the compounds of formula (Ia), (Ib), (Ic) or (Id), respectively in the form of the trans (4R,5R), (4S,5S) or cis (4R,5S), (4S,5R) diastereoisomers, which if appropriate are salified,

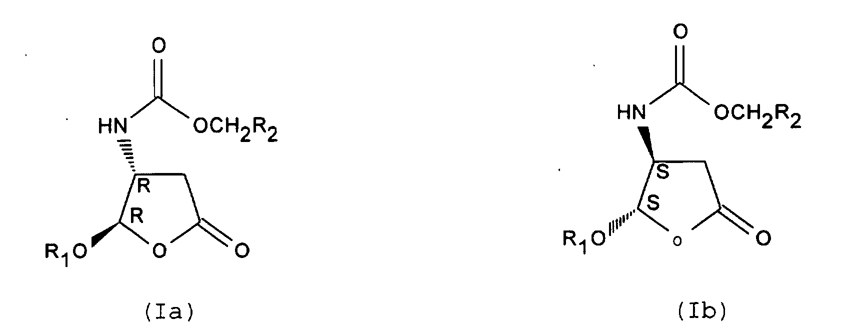

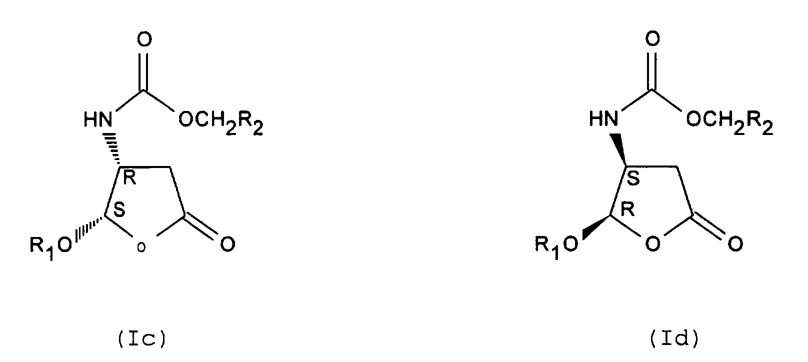

6. Process according to claim 4, **characterized in that** the separation by crystallization of the compounds of formulae (IIIa) and (IIIb) as defined in claim 4, is carried out:
a) by the action of trichloroacetic acid, in order to obtain the corresponding salt of formula (IIIa) or (IIIb),
b) then by the action of monochloroacetic acid on the mother liquors in order to obtain the salt corresponding to the other diastereoisomer of formula (IIIa) or (IIIb).

7. Preparation process according to claim 4, for the compounds of formula (IVd) as defined in claim 2 or for the compounds of formula (Id) as defined in claim 5, **characterized in that** it includes at least one of the following stages:
a) the action of the phenylethylamine R on the compound of formula (II), in order to obtain the following compounds of formulae (III'a) and (III'b): 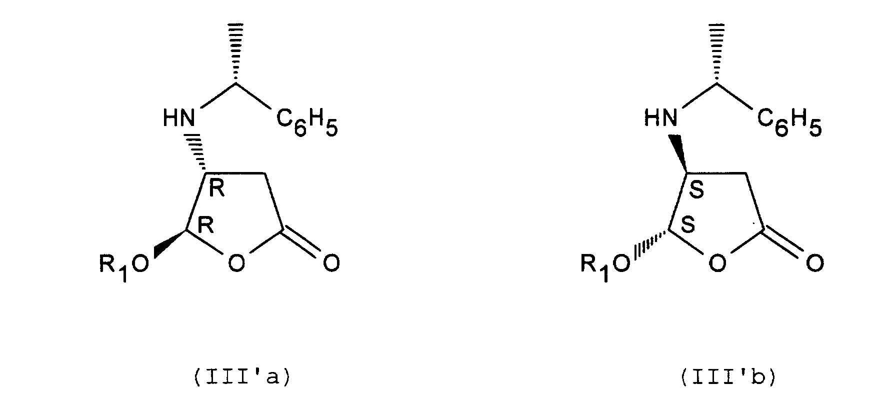
b) resolution of the trans stereoisomers of formulae (III'a) and (III'b) by the action of trichloroacetic acid in order to obtain the (4S,5S) stereoisomer (III'b) in the form of the trichloroacetic acid salt then by the action of monochloroacetic acid, in order to obtain the (4R,5R) stereoisomer (III'a) in the form of the monochloroacetic acid salt,
c) if appropriate, returning to the free amine by the action of a base,
d) epimerization reaction of the (4S,5S) stereoisomer of formula (III'b) in the presence of an acid in order to obtain a cis (4S,5R) stereoisomer of formula (III'd): 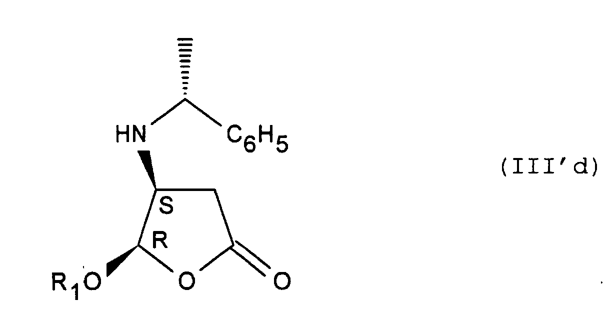
e) if appropriate, crystallization after salification by the action of monochloroacetic or dichloroacetic acid,
f) if appropriate, returning to the free amine by the action of a base,
g) if appropriate, recrystallization after salification, in particular, in the form of the hydrochloride,
h) hydrogenolysis of the cis (4S,5R) stereoisomer of formula (III'd), in order to obtain the compound of formula (IVd) in the form of the cis (4S,5R) diastereoisomer,
i) if appropriate, the action of the allyl chloroformate on the compound of formula (IVd) in order to obtain the compound of formula (Id) in the form of the cis (4S,5R) diastereoisomer with R2 representing -CH=CH₂.

8. Preparation process according to claim 4, for the compounds of formula (IVd) as defined in claim 2 or for the compounds of formula (Id), as defined in claim 5, **characterized in that** it includes at least one of the following stages:
a) the action of the phenylethylamine S on the compound of formula (II), in order to obtain the following compounds of formulae (III"a) and (III"b): 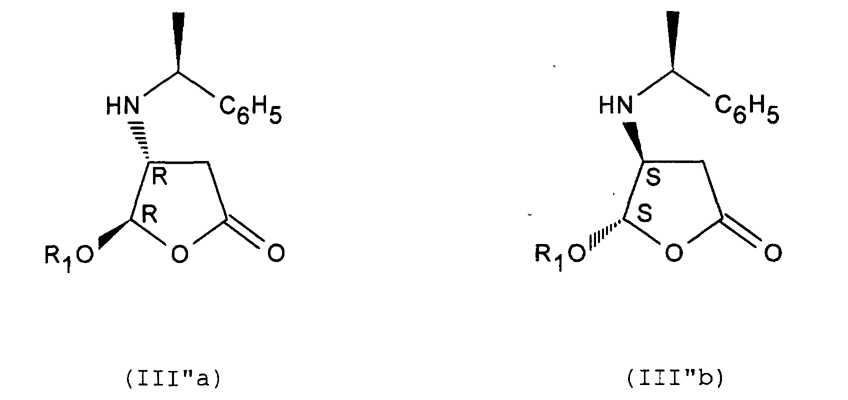
b) resolution of the trans stereoisomers of formulae (III"a) and (III"b) by the action of trichloroacetic acid in order to obtain the (4R,5R) stereoisomer (III"a) in the form of the trichloroacetic acid salt then by the action of monochloroacetic acid, in order to obtain the (4S,5S) stereoisomer (III"b) in the form of the monochloroacetic acid salt,
c) if appropriate, desalification by the action of a base,
d) epimerization reaction of the (4S,5S) stereoisomer of formula (III"b) in the presence of an acid in order to obtain the cis (4S,5R) stereoisomer of formula (III"d): 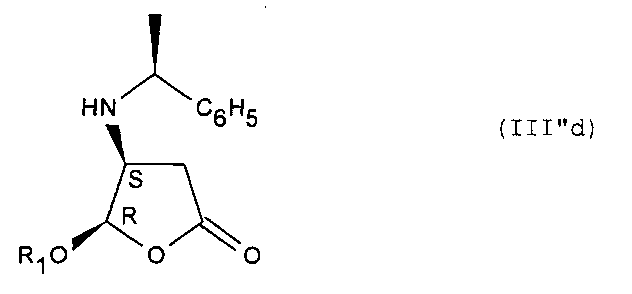
e) if appropriate, crystallization after salification by the action of an acid such as monochloroacetic or dichloroacetic acid,
f) if appropriate, returning to the free amine by the action of a base,
g) if appropriate, recrystallization after salification in particular in the form of the hydrochloride,
h) hydrogenolysis of the cis (4S,5R) stereoisomer of formula (III"d), in order to obtain the compound of formula (IVd) in the form of the cis (4S,5R) diastereoisomer
i) if appropriate, the action of the allyl chloroformate on the compound of formula (IVd) in order to obtain the compound of formula (Id) in the form of the cis (4S,5R) diastereoisomer, R₂ representing a -CH=CH₂ radical.

9. Process as defined in any one of claims 4 to 8 **characterized in that** the addition of the amine on the compound of formula (II) as defined in claim 4, is carried out in dimethylformamide or aqueous isopropanol.

10. Process as defined in any one of claims 4 to 8 **characterized in that** the epimerization reaction is carried out with tin tetrachloride or methane sulphonic acid.

11. Process as defined in any one of claims 4 to 8 **characterized in that** R₁ is an ethyl radical.

12. Process as defined in any one of claims 8 to 11, **characterized in that** the resolution of the trans stereoisomers with trichloroacetic acid (Stage b) is carried out in aqueous isopropanol.

13. Process as defined in any one of claims 8 to 11, **characterized in that** the epimerization reaction of the (4S,5S) stereoisomer of formula (III"b, Stage d) is carried out in the presence of methane sulphonic acid in toluene.

14. Process as defined in any one of claims 8 to 11, **characterized in that** the crystallization (Stage e) is carried out by the action of dichloroacetic acid in toluene.

15. Use:
either of the compounds of formula (IVa), (IVb), (IVc) or (IVd) as defined in claim 1 or originating from the process as defined in claim 4,
or of the compounds of formula (Ia), (Ib), (Ic) or (Id) originating from the process as defined in claim 5 in amidification reactions starting from the acid of formula A-COOH in order to obtain a following compound of formula (V) : 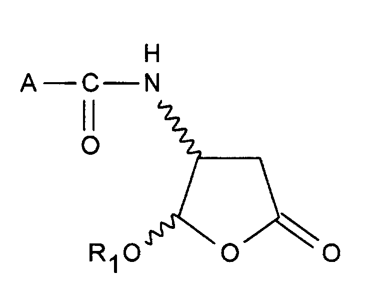 A representing any organic radical.

16. Use according to claim 15:
either of the compounds of formula (IVb) or (IVd) as defined in claim 1 or originating from the process as defined in claim 4,
or of the compounds of formula (Ib) or (Id) originating from the process as defined in claim 5 in the synthesis of the compounds of formula (V) having an inhibitory activity on the interleukin converting enzyme.

17. Use according to claim 15:
either of the compound of formula (IVd) with R₁ = ethyl, as defined in claim 3 or originating from the process as defined in claim 7 or 8,
or of the compound of formula (Id) with R₁ = ethyl, originating from the process as defined in claim 5 for the preparation of a compound of formula (V) having an inhibitory activity on the interleukin converting enzyme.

18. Use according to claim 17 of the compounds of formula (IVd) or (Id) as defined in claim 17, for the preparation of the compound of formula (V) having the following structure:

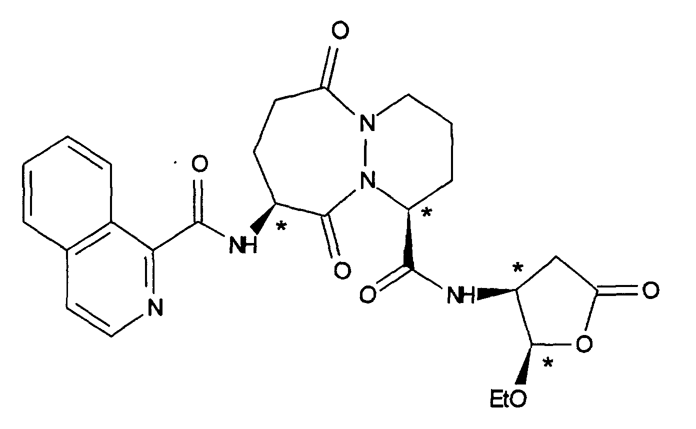

19. Compounds of formulae (IIIa), (IIIb), (IIIc), (IIId), as new intermediate compounds, as well as their addition salts with acids, with the exception of the compounds of formulae (III"a) and (III"b) as defined in claim 8 with R₁ = methyl.

## Patentansprüche

1. Verbindungen der folgenden Formel (IVa), (IVb), (IVc) oder (IVd): 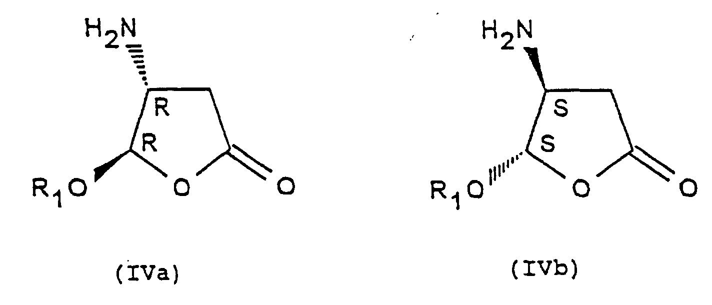 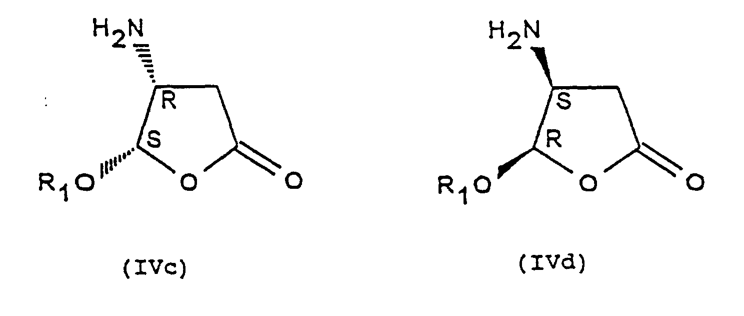 in denen R₁ eine Alkyl-Gruppe mit 1 bis 4 Kohlenstoffatomen oder eine Phenylalkyl-Gruppe mit 7 bis 11 Kohlenstoffatomen ist,
sowie ihre Additionssalze mit Säuren.

2. Verbindungen der Formel (IVd) nach Anspruch 1 sowie ihre Additionssalze mit Säuren.

3. Verbindungen der Formel (IVd) nach Anspruch 2, wobei R₁ eine Ethyl-Gruppe ist, sowie ihre Additionssalze mit Säuren.

4. Verfahren zur Herstellung der Verbindungen der Formel (IVa), (IVb), (IVc) oder (IVd) nach Anspruch 1, **dadurch gekennzeichnet, daß** es mindestens eine der folgenden Stufen umfaßt:
a) Umsetzung eines Arylamins der Formel R₃R₄CHNH₂, in der R₃ ein Wasserstoffatom oder eine Alkyl-Gruppe mit 1 bis 4 Kohlenstoffatomen ist und R₄ ein gegebenenfalls substituiertes Aryl ist, mit racemischem Alkyloxyfuranon der Formel (II): 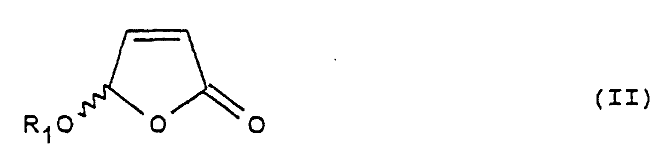 in der R₁ eine Alkyl-Gruppe mit 1 bis 4 Kohlenstoffatomen oder eine Phenylalkyl-Gruppe mit 7 bis 11 Kohlenstoffatomen ist, und
Erhalt der isomeren trans-(4R,5R)- und trans-(4S,5S)-Verbindungen der Formel (IIIa) bzw. (IIIb): 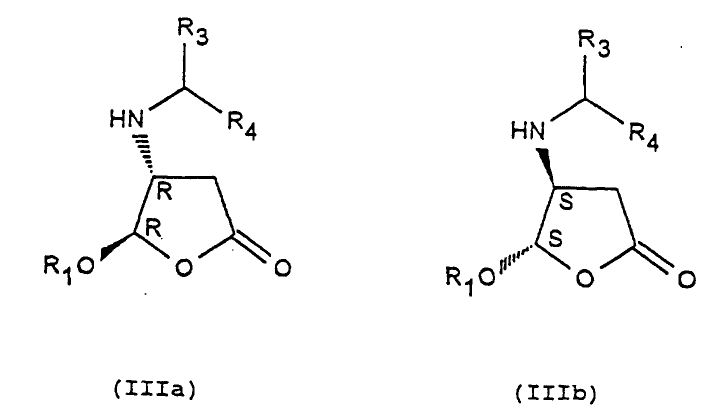 die dann
- durch Kristallisation getrennt werden, indem gegebenenfalls eine oder mehrere Salzbildungsreaktion(en) durchgeführt wird (werden), oder
- wenn R₃ ein Wasserstoffatom ist, durch Wirkung einer optisch aktiven Säure getrennt werden;
b) gegebenenfalls Epimerisierungsreaktion der Verbindung der Formel (IIIa) oder (IIIb), die in ein Salz übergeführt ist oder nicht, in Gegenwart einer Säure, um die cis-(4R,5S)- oder (4S,5R)-Isomeren der Formel (IIIc) oder (IIId), die gegebenenfalls in ein Salz übergeführt sind, zu erhalten; 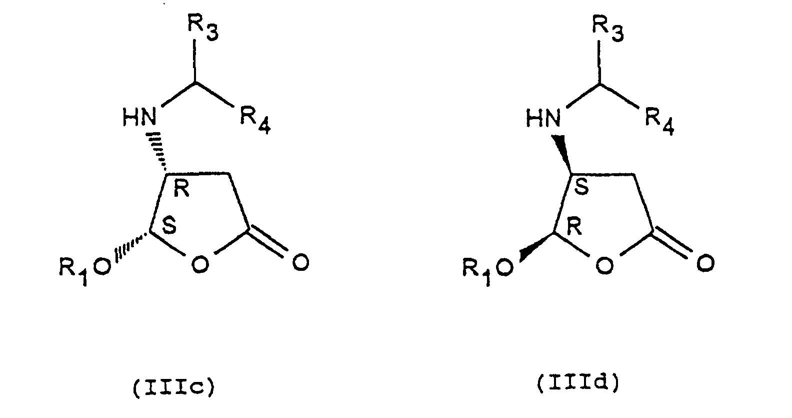
c) Hydrogenolysereaktion
- entweder mit dem trans-(4R,5R)- oder trans-(4S,5S)-Isomeren der Formel (IIIa) oder (IIIb), das in ein Salz übergeführt ist oder nicht, um die Verbindung der Formel (IVa) oder (IVb) in Form des trans-(4R,5R)- oder trans-(4S,5S)-Isomeren, das gegebenenfalls in ein Salz übergeführt und/oder geschützt ist, zu erhalten;
- oder mit dem cis-(4R,5S)- oder cis-(4S,5R)-Isomeren der Formel (IIIc) oder (IIId), das in ein Salz übergeführt ist oder nicht, um die Verbindung der Formel (IVc) oder (IVd) in Form des cis-(4R,5S)- oder cis-(4S,5R)-Isomeren, das in ein Salz übergeführt ist und/oder geschützt ist, zu erhalten.

5. Verfahren nach Anspruch 4, **dadurch gekennzeichnet, daß** die Reaktion zum Schützen der Amine der Formel (IVa), (IVb), (IVc) oder (IVd), die in ein Salz übergeführt sind oder nicht, durch Umsetzung mit Chlorformiat der Formel Cl-CO-O-CH₂-R₂ erfolgt, worin R₂ eine tert-Butyl-, (C₂-C₄)-Alkenyl-, (C₂-C₄)-Alkinyl- oder PhenylGruppe, die substituiert oder nicht-substituiert ist, darstellt, wobei die Verbindungen der Formel (Ia), (Ib), (Ic) oder (Id) jeweils in Form der trans-(4R,5R)-, trans-(4S,5S)- oder cis-(4R,5S)-, cis-(4S,5R)-Diastereoisomeren, die gegebenenfalls in Salze überführt sein können, erhalten werden.

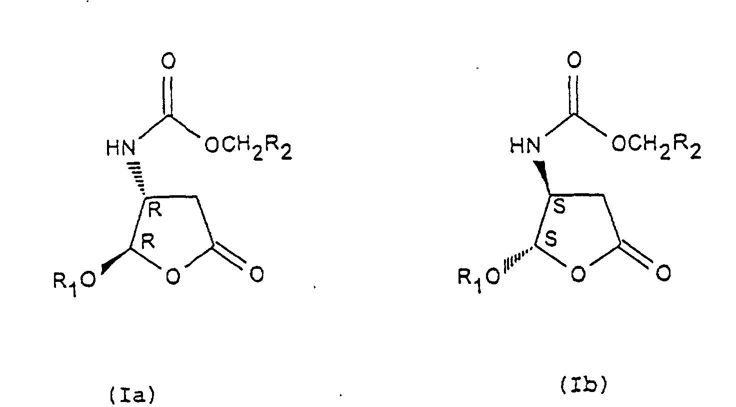

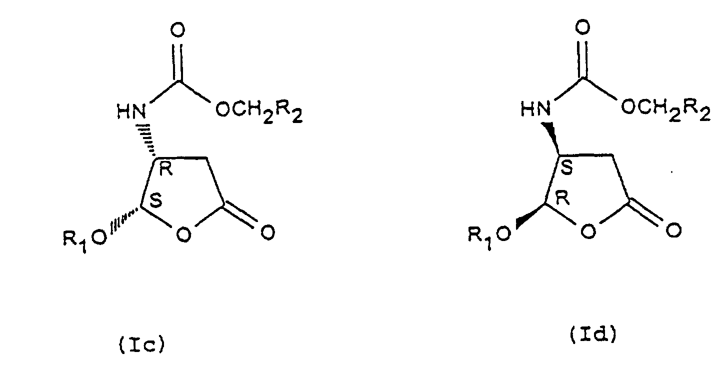

6. Verfahren nach Anspruch 4, **dadurch gekennzeichnet, daß** die Trennung durch Kristallisation der Verbindungen der Formeln (IIIa) und (IIIb), wie sie in Anspruch 4 definiert sind, wie folgt durchgeführt wird:
a) durch Umsetzung mit Trichloressigsäure unter Erhalt des Salzes, das Formel (IIIa) oder (IIIb) entspricht,
b) dann Einwirkung von Monochloressigsäure auf die Mutterlauge, um das Salz zu erhalten, das dem anderen Diastereomeren der Formel (IIIa) oder (IIIb) entspricht.

7. Verfahren nach Anspruch 4 zur Herstellung der Verbindungen der Formel (IVd), wie sie in Anspruch 2 definiert sind, oder der Verbindungen der Formel (Id), wie sie in Anspruch 5 definiert sind, **dadurch gekennzeichnet, daß** es mindestens eine der folgenden Stufen umfaßt:
a) Umsetzung des Phenylethylamins R mit der Verbindung der Formel (II) unter Erhalt der Verbindungen der folgenden Formeln (III'a) und (III'b): 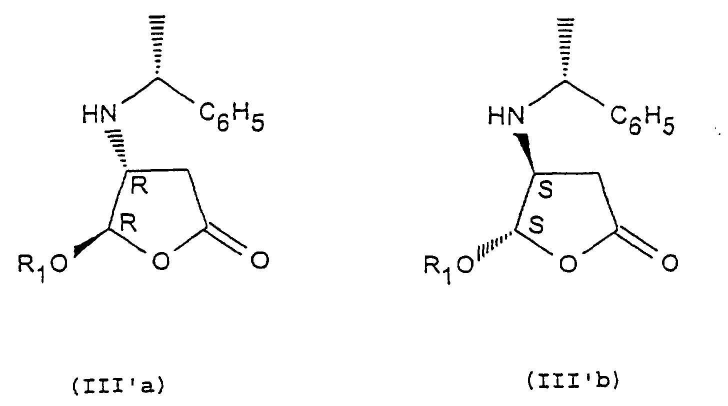
b) Trennung der trans-Stereoisomeren der Formeln (III'a) und (III'b) durch Einwirkung von Trichloressigsäure, um das (4S,5S)-Stereoisomer (III'b) in Form des Salzes von Trichloressigsäure zu erhalten, danach Einwirkung von Monochloressigsäure, um das (4R,5R)-Stereoisomer (III'a) in Form des Salzes mit Monochloressigsäure zu erhalten,
c) gegebenenfalls Rückumwandlung in das freie Amin durch Wirkung einer Base,
d) Epimerisierungsreaktion des (4S,5S)-Stereoisomeren der Formel (III'b) in Gegenwart einer Säure, um ein cis-(4S,5R)-Stereoisomer der Formel (III'd) zu erhalten: 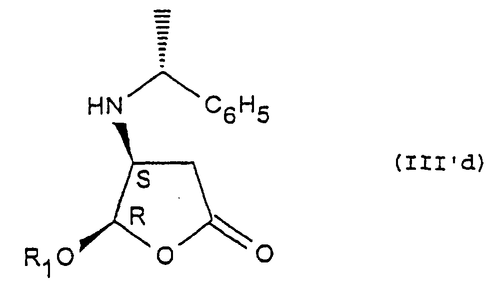
e) Kristallisation nach Salzbildung durch Wirkung von Monochloressigsäure oder Dichloressigsäure,
f) gegebenenfalls Rückumwandlung in das freie Amin durch Wirkung einer Base,
g) gegebenenfalls Umkristallisation nach Salzbildung, insbesondere in Form des Hydrochlorids,
h) Hydrogenolyse des cis-(4S,5R)-Stereoisomeren der Formel (III'd), um die Verbindung der Formel (IVd) in Form des cis-(4S,5R)-Diastereomeren zu erhalten,
i) gegebenenfalls Einwirkung von Allylchlorformiat auf die Verbindung der Formel (IVd), um die Verbindung der Formel (Id) in Form des cis-(4S,5R)-Diastereomeren,
worin R2 -CH=CH₂ darstellt, zu erhalten.

8. Verfahren nach Anspruch 4 zur Herstellung der Verbindungen der Formel (IVd), wie sie in Anspruch 2 definiert sind, oder der Verbindungen der Formel (Id), wie sie in Anspruch 5 definiert sind, **dadurch gekennzeichnet, daß** es mindestens eine der folgenden Stufen umfaßt:
a) Umsetzung des Phenylethylamin S mit der Verbindung der Formel (II) unter Erhalt der Verbindungen der Formeln (III"a) und (III"b): 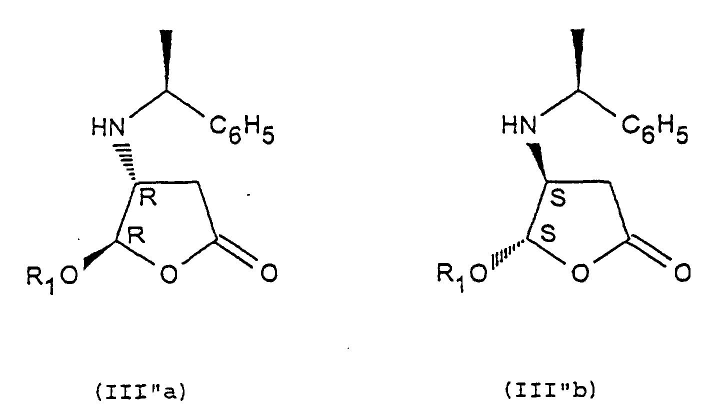
b) Trennung der trans-Stereoisomeren der Formeln (III"a) und (III"b) durch die Wirkung von Trichloressigsäure, um das (4R,5R)-Stereoisomer (III"a) in Form der Salzes von Trichloressigsäure zu erhalten, danach Einwirkung von Monochloressigsäure, um das (4S,5S)-Stereoisomere (III"b) in Form des Salzes von Monochloressigsäure zu erhalten,
c) gegebenenfalls Entsalzung durch Einwirkung einer Base,
d) Epimerisierungsreaktion des (4S,5S)-Stereoisomeren der Formel (III"b) in Gegenwart einer Säure, um das cis-(4S,5R)-Stereoisomer der Formel (III"d) zu erhalten: 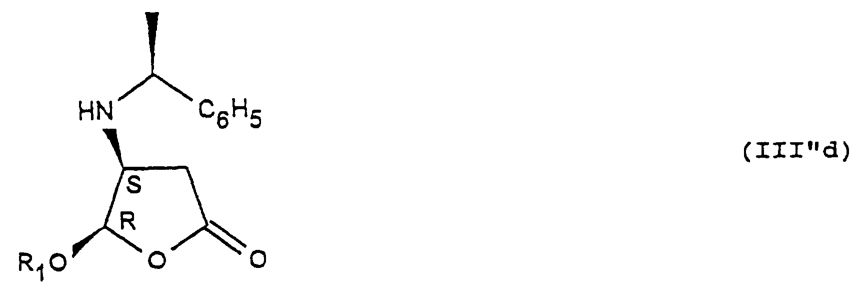
e) gegebenenfalls Kristallisation nach Salzbildung durch Einwirkung einer Säure wie Monochloressigsäure oder Dichloressigsäure,
f) gegebenenfalls Zurückführung zum freien Amin durch Wirkung einer Base,
g) gegebenenfalls Umkristallisation nach Salzbildung, insbesondere in Form des Hydrochlorids,
h) Hydrogenolyse des cis-(4S,5R)-Stereoisomeren der Formel (III"d), um die Verbindung der Formel (IVd) in Form des cis-(4S,5R)-Diastereomeren zu erhalten,
i) gegebenenfalls Einwirkung des Allylchloroformiats auf die Verbindung der Formel (IVd), um die Verbindung der Formel (Id) in Form des cis-(4S,5R)-Diastereomeren,
worin R₂ eine Gruppe -CH=CH₂ darstellt, zu erhalten.

9. Verfahren nach einem der Ansprüche 4 bis 8, **dadurch gekennzeichnet, daß** die Addition des Amins an die Verbindung der Formel (II), wie sie in Anspruch 4 definiert ist, in Dimethylformamid oder wäßrigem Isopropanol durchgeführt wird.

10. Verfahren nach einem der Ansprüche 4 bis 8, **dadurch gekennzeichnet, daß** die Epimerisierungsreaktion mit Zinntetrachlorid oder Methansulfonsäure durchgeführt wird.

11. Verfahren nach einem der Ansprüche 4 bis 8, **dadurch gekennzeichnet, daß** R₁ eine Ethyl-Gruppe ist.

12. Verfahren nach einem der Ansprüche 8 bis 11, **dadurch gekennzeichnet, daß** die Trennung der trans-Stereoisomeren mit Trichloressigsäure (Stufe b) in wäßrigem Isopropanol durchgeführt wird.

13. Verfahren nach einem der Ansprüche 8 bis 11, **dadurch gekennzeichnet, daß** die Epimerisierungsreaktion des (4S,5S)-Stereoisomeren der Formel (III"b, Stufe d) in Gegenwart von Methansulfonsäure in Toluol durchgeführt wird.

14. Verfahren nach einem der Ansprüche 8 bis 11, **dadurch gekennzeichnet, daß** die Kristallisation (Stufe e) durch die Wirkung von Dichloressigsäure in Toluol erfolgt.

15. Verwendung
der Verbindungen der Formel (IVa), (IVb), (IVc) oder (IVd) nach Anspruch 1 oder erhalten aus dem Verfahren nach Anspruch 4,
oder der Verbindungen der Formel (Ia), (Ib), (Ic) oder (Id) aus dem Verfahren nach Anspruch 5 in Amidierungsreaktionen, ausgehend von.einer Säure der Formel A-COOH unter Erhalt einer Verbindung der folgenden Formel (V): 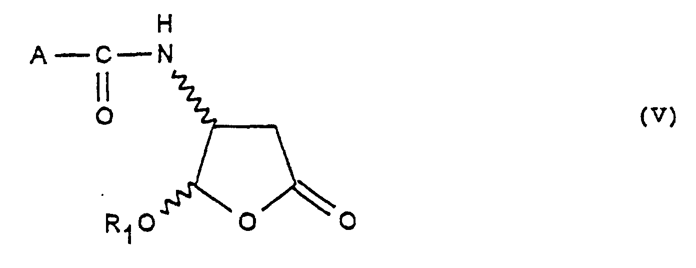 worin A irgendeinen organischen Rest darstellt.

16. Verwendung nach Anspruch 15
der Verbindungen der Formel (IVb) oder (IVd) nach Anspruch 1 oder erhalten aus dem Verfahren nach Anspruch 4 oder
die Verbindungen der Formel (Ib) oder (Id), erhalten nach dem Verfahren nach Anspruch 5, in der Synthese der Verbindungen der Formel (V), die Inhibitor-Aktivität für das Umwandlungsenzym von Interleukin besitzen.

17. Verwendung nach Anspruch 15
der Verbindung der Formel (IVd), worin R₁ = Ethyl, nach Anspruch 3 oder erhalten nach dem Verfahren nach Anspruch 7 oder 8, oder
der Verbindung der Formel (Id), worin R₁ = Ethyl, erhalten nach dem Verfahren gemäß Anspruch 5,
bei der Herstellung einer Verbindung der Formel (V), die Inhibitoraktivität für das Umwandlungsenzym von Interleukin hat.

18. Verwendung nach Anspruch 17 der Verbindungen der Formel (IVd) oder (Id) nach Anspruch 17 bei der Herstellung der Verbindung der Formel (V), die die folgende Struktur hat:

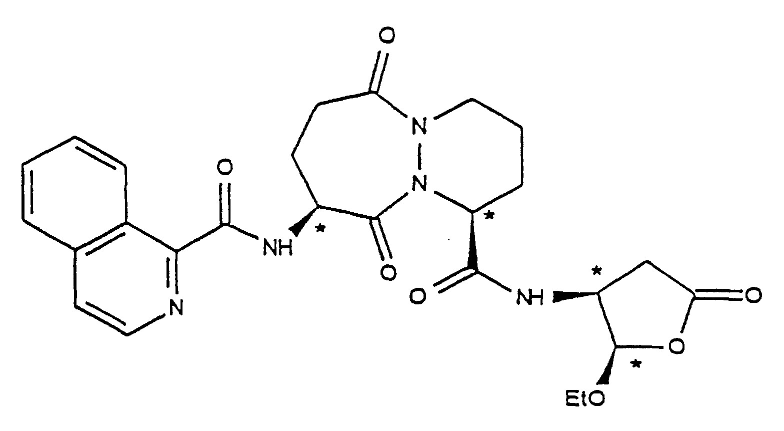

19. Verbindungen der Formel (IIIa), (IIIb), (IIIc), (IIId) als neue Zwischenverbindungen sowie ihre Additionssalze mit Säuren,ausgenommen die Verbindungen der Formeln (III"a) und (III"b), wie sie in Anspruch 8 definiert sind, wobei R₁ = Methyl ist.
